# EUROPEAN PATENT APPLICATION

(11) **EP 4 454 634 A1**
(43) Date of publication of application: **30.10.2024**
(21) Application number: 21968855.3
(22) Date of filing: 21.12.2021
(51) Int. Cl.: A61K 8/04, A61K 8/27, A61K 8/29, A61K 8/39, A61K 8/86

(54) **AQUEOUS DISPERSION AND COSMETIC PREPARATION**

(71) Applicant: Miyoshi Kasei, Inc., Tokyo 102-0073 (JP)
(72) Inventor: HASEGAWA, Yukio, Tokyo 102-0073 (JP)
(74) Representative: Glawe, Delfs, Moll
(86) International application number: PCT/JP2021/047313
(87) International publication number: WO 2023/119418

(57) **Abstract**

Provided is an aqueous dispersion, containing at least 3 components of (A) polyoxyethylene (5 to 15) isostearyl ether, (B) water, and (C) hydrophobized inorganic powder. The aqueous dispersion has an excellent long term dispersion stability and excellent suitability in use .

## Description

### FIELD

The present invention relates to an aqueous dispersion and a cosmetic. Particularly, the present invention relates to an aqueous dispersion of a hydrophobized inorganic powder. In detail, [the present invention] relates to an aqueous dispersion of a hydrophobized inorganic powder containing at least three components of (A) polyoxyethylene (5 to 15) isostearyl ether, (B) water and (C) hydrophobized inorganic powder, which has an excellent long-term storage dispersion stability and usability capable of satisfying both acceleration tests and cycle tests. The aqueous dispersion of the present invention relates to an excellently usable aqueous dispersion in which incompatible components, i.e., water and hydrophobized inorganic powder, are uniformly dispersed in aqueous phase. By virtue of containing the aqueous dispersion in a cosmetic, the cosmetic provides a fresh use feeling, an excellent use feeling of the hydrophobized inorganic powder, a uniform finish and an excellent UV shielding effect, thus there is provided a cosmetic having better cosmetic effects and cosmetic durability.

### BACKGROUND

When a cosmetic contains water, it is prospected that the cosmetic provides a fresh use feeling, a moisture feeling and a refreshing feeling upon evaporation of water. In addition, water is a component desired to be contained in cosmetics as much as possible since water is an essential component of living organisms and also has an extremely high safety. Inorganic powder is hydrophilic, thus easily compatible with water. However, the inorganic powder has an ionic charge due to its chemical composition, thus it is electrically charged. pH useable in cosmetic, which has a range from a weak acidic level to a weak basic level, includes a pH range at which ionic properties are reversed and results in occurrence of aggregation. Therefore, when powders with different chemical compositions are mixed, they suffer from aggregation and provide precipitations since dispersion stability cannot be maintained, resulting in failure in performing their powder performances. In addition, inorganic powders that are dispersed in water but not hydrophobized, has a tendency of providing inorganic particle specific-physical irritation characteristics upon application to skin and after application. In order to realize preferable sensory characteristics, such as finish and use feeling, as cosmetics containing inorganic powder in its aqueous phase, it is preferable to contain inorganic powder which has been hydrophobized with an organic compound, thus the sensory characteristics have a possibility of being improved. However, the hydrophobized inorganic powder is water-repellent, thus when water and the hydrophobized inorganic powder are mixed, not only they do not provide a dispersion, but also they result in aggregation of the hydrophobized inorganic powder due to hydrophobic interaction. Recently, various technologies are disclosed in which aqueous cosmetics contain the hydrophobized inorganic powder. (PTL (Patent Literatures) 1 to 3) In addition, a technology relating to an aqueous dispersion is also disclosed, in which zeta potential is controlled by containing ionic polymers. (PTL 4)

### CITATION LIST

### PATENT LITERATURE

PTL 1: WO2013/18827A
PTL 2: Tokkai JP 2015-78243 A
PTL 3: Tokkai JP 2015-203026 A
PTL 4: JP 4060849 B

### SUMMARY

### TECHNICAL PROBLEM

However, a composition such as an aqueous dispersion in which hydrophobized inorganic powder is dispersed in aqueous phase has a difficulty in preservation as a stock if it does not have an ensured long-term storage stability, resulting in deterioration in product value as an intermediate material. In [a field of] the aqueous dispersion in which the hydrophobized inorganic powder is dispersed in its aqueous phase, there is no known aqueous dispersion having an excellent long-term storage dispersion stability satisfying both acceleration tests and cycle tests and excellent suitability in use, thus there is a problem to be solved. Further, above conventional aqueous dispersions are still not enough to provide fresh use feeling of water, excellent use feeling of hydrophobized inorganic powder, uniform finish, coloring property, texture having transparency, excellent UV shielding effect, better cosmetic effect and cosmetic durability.

### SOLUTION TO PROBLEM

The present invention has been made under consideration of the above problem, and relates to a composition containing at least 3 components of (A) polyoxyethylene (5 to 15) isostearyl ether, (B) water and (C) hydrophobized inorganic powder, which has an excellent long-term storage dispersion stability and an excellent suitability in use . It was found that a cosmetic containing the aqueous dispersion may further improve fresh use feeling, excellent use feeling of the hydrophobized inorganic powder, coloring property, transparency, UV shielding effect and the like.

That is, according to a first aspect of the present invention, there is provided an aqueous dispersion, containing at least 3 components of
(A) polyoxyethylene (5 to 15) isostearyl ether,
(B) water, and
(C) hydrophobized inorganic powder,
wherein the aqueous dispersion has an excellent dispersion stability and excellent suitability in use satisfying a cycle test: -20°C to 40°C/ 2 weeks, a cycle test: 5°C to 60°C/ 2 weeks, an acceleration test: 40 ± 2°C/75%RH± 5%RH/6 months, and
(C) the hydrophobized inorganic powder comprises any one or more of titanium oxide, zinc oxide, or iron oxide having a primary particle size of 3µm or less and wherein the aqueous dispersion has a content ratio of the hydrophobized inorganic powder of 40wt% or more.

In the first aspect, it is preferable that the hydrophobized inorganic powder as the component (C) comprises an inorganic powder treated with one or more hydrophobic agents: silicone compounds, fatty acids, acylated amino acids, hydrogenated lecithin, ester oils, alkyl silanes, and alkyl phosphate.

In the first aspect, it is preferable that the polyoxyethylene (5 to 15) isostearyl ether as the component (A) is polyoxyethylene (10) isostearyl ether.

According to a second aspect of the present invention, there is provided a cosmetic, containing the aqueous dispersion according to the first aspect.

According to further aspects of the present invention, there is provided a specific producing method of the aqueous dispersion and cosmetic, and there is further provided the aqueous dispersion and cosmetic produced by the specific producing method.

### ADVANTAGEOUS EFFECTS OF INVENTION

According to the present invention, the cosmetic containing the aqueous dispersion containing at least 3 components of (A) polyoxyethylene (5 to 15) isostearyl ether, (B) water, and (C) hydrophobized inorganic powder, and having an excellent long-term storage stability and usability may further improve fresh use feeling, excellent usability of the hydrophobized inorganic powder, coloration, transparency, UV shielding ability, etc. Further aspects, forms, effects, etc. of the present invention are also apparent from the entire description of the present application (including claims, embodiments, and examples).

### BRIEF DESCRIPTION OF THE DRAWINGS

Figure 1 is a chart of zeta potential of aqueous dispersion of Example 2.
Figure 2 is a chart of zeta potential of aqueous dispersion of Example 5.
Figure 3 is a chart of zeta potential of aqueous dispersion of Example 6.
Figure 4 is a chart of zeta potential of aqueous dispersion of Example 7.
Figure 5 is a chart of zeta potential of aqueous dispersion of Example 9.
Figure 6 is a chart of zeta potential of aqueous dispersion of Example 11.
Figure 7 is a chart of zeta potential of aqueous dispersion of Example 15.
Figure 8 is a chart of zeta potential of aqueous dispersion of Example 16.
Figure 9 is a chart of zeta potential of aqueous dispersion of Example 17.
Figure 10 is a chart of zeta potential of aqueous dispersion of Example 18.

### MODES

The present invention is explained in detail as follows.

### (Component (A): polyoxyethylene (5 to 15) isostearyl ether)

The hydrophilic surfactant contained in the aqueous dispersion of the present invention is polyoxyethylene (5 to 15) isostearyl ether. "(5 to 15)" refers to added mole number of polyoxyethylene [length of polyoxyethylene chain]. It is a monoether compound of isostearyl alcohol and 5 to 15 moles of polyoxyethylene [polyoxyethylene comprising 5 to 15 moles of oxyethylene]. Isostearyl alcohol as a hydrophobic group is a branched alcohol having 18 carbons. Branching types include monomethyl-branched type, dimethyl-branched type, garbetted type, and multi-branched type, but any one of these types is acceptable. An effect of the present invention is not exerted by a monoether compound having straight-chain 18-carbon alcohol or alcohols other than 18 carbons. An effect of the present invention is not exerted by a diether type of isostearyl alcohols and polyoxyethylene.

The polyoxyethylene (5-15) isostearyl ether of the present invention may make hydrophobized inorganic powder to be easily and stably mixed with and dispersed in water or an aqueous phase so as to provide an aqueous dispersion. Polyoxyethylene (5-15) isostearyl ether of the present invention is an essential component in the aqueous dispersion for obtaining long-term storage dispersion stability and excellent suitability in use in terms of acceleration tests and cycle tests.

A generally available polyoxyethylene (5-15) isostearyl ether that may achieve an effect of the present invention is exemplified by EMALEX 1805 (Nippon Emulsion Co., Ltd.), Nonion IS-205 (Nichiyu Oil Co., Ltd.). Ltd.), etc., as a monoether of 5 mol-polyoxyethylene [polyoxyethylene comprising 5 moles of oxyethylene], EMALEX 1810 (Nippon Emulsion Co., Ltd.), Nonion IS-210 (Nichiyu Oil Co., Ltd.), etc., as a monoether of 10 mol-polyoxyethylene, and EMALEX 1815 (Nippon Emulsion Co., Ltd.), Nonion IS-215 (Nichiyu Co., Ltd.), etc., as a monoether of 15 mol-polyoxyethylene. These polyoxyethylene isostearyl ethers may be used in combination.

### (Component (B): water)

Water referred in the present invention is ion-exchanged water, distilled water, etc., that may be used for cosmetics. In the present invention, a system containing water as a main component and at least another component compatible or miscible with water is referred to as aqueous components. As components other than water capable of being contained as the aqueous components are exemplified by alcohols, for example, ethanol, hexanediol, pentanediol, benzyl alcohol, phenoxyethanol, propylene glycol, dipropylene glycol, 1,3 butylene glycol, polyethylene glycol, isopentyl diol, caprylyl glycol, glycerin, diglycerin, polyglycerin, ethylhexylglycerin, hexylglycerin, cyclohexylglycerin, trimethylolpropane, xylitol, erythritol, trehalose, sorbitol, etc. One or more of these components may be contained. These components may be contained within an extent that they do not impair the long-term aging stability of the dispersion of the present invention.

Optional components other than the alcohols that may be contained are exemplified by water-soluble polymers, organic thickeners, inorganic thickeners and organically treated inorganic thickeners as dispersion stabilizers, as well as, for example, UV absorbers, swelling agents, moisturizers, emollients, antibacterial agents, preservatives, fragrances, antioxidants, coolants, anti-inflammatory agents, skin beauty compositions, skin astringents, vitamins, etc., if another effects or functions are desired. As antimicrobial emollients, the isopentyl diol, caprylyl glycol, phenethyl alcohol, phenylpropanol, glyceryl monocaprylate, etc., may be contained. These optional components may be contained in water as the component (B) of the aqueous dispersion of the present invention at 10wt% or less. It is preferable that the optional components do not comprise components having anionic or cationic properties when they are miscible with or mixed in water. If chelating agents that blocks ions in water are contained, stability over time is significantly worsen. Mixing refers to the other components are dissolved in or mixed with water, and the other components may be liquid or solid form at room temperature. It is preferable that a dispersion stabilizer is contained as an agent to prevent particle sedimentation under a case that inorganic particles have a primary particle size of submicron or larger. As the dispersion stabilizer, polyvinylpyrrolidone, organic bentonite, partially hydrophobized cellulose, ethyl cellulose, CNF (cellulose nanofiber), etc. may be contained.

Recently, such cosmetics are also developed by cosmetic industry, that do not use precious drinkable water, in response to UNESCO's prediction that about 47% of the world's population will face water shortages by 2030. So-called sustainable water materials that have been developed in response to such situation are exemplified by sake lees water (Sake Lees Water (HD2): Radiant, Inc.), rice fermentation liquid (Horus Ginjo Extract AL: Horus Co.) and the like. They are water products of water recovered by distillation or extraction from sake lees after sake brewing, thus they contain very small amounts of minerals and amino acids as other components. Such water products may be used as water of the dispersion even though they contain miscellaneous ions above.

In addition, in order to improve feeling upon aqueous dispersion is applied to skin, oily components may be contained within an extent that the aqueous dispersion does not lose its stability over time. The oily components are exemplified by, for example, non-volatile hydrocarbon oils such as squalane, liquid paraffin, etc., as well as ester oils such as cetyl ethylhexanoate, cetearyl isononanoate, isoamyl laurate, hexyl laurate, decyl laurate, dicaprylyl ether, isopropyl myristate, 2-hexyl decyl myristate, octyldodecyl myristate, 2-ethylhexyl palmitate, cetyl palmitate, 2-hexyldecyl stearate, ethyl isostearate, 2-hexyldecyl isostearate, isostearyl isostearate, phenoxyethyl caprylate, alkyl benzoate (C12-C15), isotridecyl isononanoate, methyl pentanediol dinopentanoate, diethyl hexyl succinate, diisopropyl adipate, diethyl hexyl adipate, diethyl hexyl sebacate, tri(caprylic/capric)glyceryl, shea oil, almond oil, avocado oil, olive oil, etc. They may be contained at 3% or less of the total amount of the aqueous dispersion.

### (Component (C): hydrophobized inorganic powder)

The hydrophobized inorganic powder used in the present invention is inorganic powder having hydrophobicity which is covered with organic surface treatment agent as a hydrophilizing agent.

The organic surface treatment agent for the hydrophobized inorganic powder in the present invention is exemplified by one or more compounds selected from silicone compounds, acylated amino acids, fatty acids, lecithin, ester oils, alkyl silanes, alkyl phosphates, organic titanates, and fructo-oligosaccharides.

As the silicone compounds used in the present invention, may be used are methylhydrogen polysiloxane (Shin-Etsu Chemical: KF99P and KF9901, X-24-9171, X-24-9221, etc.), dimethiconol, one-ended alkoxysilyl dimethylpolysiloxane, trimethyl siloxysilicate and cyclic methyl hydrogensiloxanes such as tetrahydrotetramethylcyclotetrasiloxane, etc., as well as acrylic silicone, silicone acryl, amino-modified silicone, carboxy-modified silicone, phosphate-modified silicone, etc. As the other silicone compounds, may be used are KF-9908 (triethoxysilyl ethyl polydimethylsiloxyethyl dimethicone), KF-9909 (triethoxysilyl ethyl polydimethylsiloxyethylhexyl dimethicone) and the like as commercially available products from Shin-Etsu Chemical.

The acylated amino acids used in the present invention include acylated compounds of saturated fatty acids having 12 to 18 carbons and amino acids selected from aspartic acid, glutamic acid, alanine, glycine, sarcosine, proline, hydroxyproline, leucine and isoleucine, or total hydrolysates of peptides derived from plants such as wheat and peas, silk peptides, peptides derived from animals, etc. Carboxyl groups of the amino acids may be free or in a form of salts of such as K, Na, Fe, Zn, Ca, Mg, Al, Zr, Ti, etc. Concretely, they are exemplified by Amisoft CS-11, CS-22, MS-11, HS-11P, HS-21P, etc., commercially available from Ajinomoto; Soipon SLP, Soipon SCA, Alanon AMP, etc., commercially available from Kawaken Fine Chemical Co.; SEPILIFT DPHP (dipalmitoyl hydroxyproline), VOLUFOAM (palmitoyl isoleucine), TIMECODE (palmitoyl glycine), etc., commercially available from SEPPIC of France; and sarcosinate MN, etc. commercially available from Nikko Chemical. These acylated amino acids may also be in a form of compositions with fatty acids. Acylated lipoamino acid composition may be exemplified by SEPIFEEL ONE (a composition composed of four components: palmitoyl proline, palmitoyl sarcosine, palmitoyl glutamic acid, and palmitic acid), commercially available from SEPPIC.

The fatty acids used in the present invention are exemplified by linear or branched saturated or unsaturated fatty acids with carbon numbers from 12 to 22, for example, fatty acids of lauryl acid, myristic acid, palmitic acid, stearic acid, oleic acid, linoleic acid, palmitoleic acid, behenic acid, lignoselynic acid, 2-ethylhexanoic acid, isotridecanoic acid, isomyristic acid, isopalmitic acid, isostearic acid, isobehenic acid, etc., or metal salts thereof with Ca, Mg, Zn, Zr, Al, Ti, etc.

The hydrogenated lecithin used in the present invention is exemplified by natural lecithin extracted from egg yolk, soybean, corn, rapeseed, sunflower, etc., and hydrogenated synthetic lecithin with an iodine value of 15 or less, which is a glyceride having a phosphoric acid group. Concretely, it is exemplified by Sunlipon 90H, commercially available from Perimondo, LLC, Phospholipon 90H commercially available from Lipoid GmbH, etc. They may be treated alone or in salt form. The salt is exemplified by water-insoluble hydrogenated lecithin metal salts with Al, Mg, Ca, Zn, Zr, Ti, etc.

The ester oils used in the present invention include ester compounds having a total carbon number of 16 or more, which may be obtained by reacting one or more kinds of alcohols having 1 to 36 carbons with one or more kinds of carboxylic acids having 1 to 36 carbons, preferably acidic ester oils having an acid value of 15 or more. Among known compounds disclosed in JP2004-51945A, concretely exemplified are Saracos MIS (isostearyl sebacate), Saracos MOD (octyldodecanol azelaic acid), Saracos 1A (adipic acid), Saracos HD (octyldodecanol dimer acid) as commercially available from the Nisshin OilliO group.

Alkylsilanes used in the present invention are exemplified by alkylalkoxysilanes. The length of alkyl chain is exemplified by 1 to 18 carbons, concretely exemplified by methyltriethoxysilane, hexyltriethoxysilane, octyltriethoxysilane, octadecyltriethoxysilane, aminopropyltriethoxysilane, etc.

Alkyl phosphoric acids used in the present invention include monoesters, diesters, and triesters of long-chain alkyl alcohols and phosphoric acids, to be exemplified by lauryl phosphoric acid, monocetyl phosphate, dicetyl phosphate, tricetyl phosphate, stearyl phosphate, C20-22 phosphoric acid, etc. K and Na salts of these alkyl phosphates may be also used. Commercial products of cetyl phosphate are exemplified by Purephos Alpha (Nikko Chemicals Co., Ltd.).

Organic titanates used in the present invention include those having a base structure of Ti(OR₁)₄, in which each R₁ is independently an alkyl group or an organic carbonyl group. Commercially available organic titanates are exemplified by isopropyl triisostearoyl titanate (Prenact TTS; Ajinomoto Fine Techno Co.), etc.

Dextrin fatty acid esters and fructooligosaccharide esters used in the present invention may be selected from esters of dextrin or fructooligosaccharides and fatty acids, and derivatives thereof. Concretely, among known compounds disclosed in JPHei5-3844A and JP2002-188024A, the dextrin fatty acid esters and fructooligosaccharide esters are exemplified by Rheopal KL, Rheopal MKL, Rheopal TT, Rheopal KE, Rheopal TL, Rheopal ISK, etc., commercially available from Chiba Flour Milling Co.

The inorganic powders used in the present invention are preferably inorganic powders having an average particle size of less than 3µm. Preferred inorganic powders include titanium dioxide, zinc oxide and iron oxide. Titanium dioxide is used for a purpose of reducing photocatalytic activity, and comprises that has been subjected to inorganic treatment with Al(OH)₃, Al₂O₃, and SiO₂ on the particle surface. The average particle size in the present invention refers to a primary particle size of inorganic powder particles before hydrophobization treatment. Such primary particle size may be a value published by each material manufacturer. Measuring methods of the primary particle size may be electron microscopy, XRD, or other methods, but the primary particle size published by the manufacturer may be used.

Preferable color pigments as the inorganic powders of the present invention are exemplified by pigment-grade titanium dioxide, pigment-grade zinc oxide, pigment-grade iron oxide, etc. Inorganic powders for sunscreen may be exemplified by titanium dioxide and zinc oxide of so-called non-nanoparticles having an average particle size larger than 100 nm, but including no particles of 100 nm or less. A generally available hydrophobized non-nano titanium dioxides are exemplified by Solaveil XTP1 (Cloda Co., Ltd.). Non-nano titanium dioxide that has not been hydrophobized includes Solaveil XTP2 (Kroda Co., Ltd.), which may be contained in the aqueous dispersion of the present invention if it is hydrophobized by existing organic surface treatment. Hydrophobized non-nano zinc oxide is exemplified by Solaveil MZP7 and Solaveil MZP8 (Kroda Co., Ltd.). Non-nano zinc oxide that has not been hydrophobized is exemplified by Solaveil MZP3 (Kroda Co., Ltd.), which may be contained in the aqueous dispersion of the present invention if it is hydrophobized like as the non-nano titanium dioxide. Other examples include fine titanium dioxide particles, fine zinc oxide particles, and fine iron oxide particles with an average particle size of 100 nm or less. As commercially available hydrophobized inorganic powders, stearic acid treated fine titanium dioxide particles are exemplified by MT-01, MT-100Z, MT-100TV, and MT-200ST (Teika Corporation), STR-100C-LF (Sakai Chemical Industry Co., Ltd.), ST-455, ST-461SA, ST-455FA (Titanium Industry Co., Ltd.), etc. Isostearic acid treated fine titanium dioxide particles are exemplified by MT-10EX, MT-150EX (Tayca Corporation), etc. Silicone-treated fine titanium dioxide particles are exemplified by MTY-02, MTY-100SAS, MT-500SAS (Tayca Corporation), STR-100C-LP, STR-100W-LP (Sakai Chemical Industry Co., Ltd.), ST-461EC (Titanium Industry Co., Ltd.), etc. Alkylsilane treated fine titanium dioxide particles are exemplified by MTX-05OTS (Tayca Corporation) and STR-100W-OTS (Sakai Chemical Industry Co., Ltd.). Isostearic acid-treated fine zinc oxide particles are exemplified by MZY-505EX (Tayca Corporation), and silicone-treated fine zinc oxide particles are exemplified by MZY-505S, MZY-303S (Tayca Corporation), FINEX-33W-LP2 (Sakai Chemical Industry Co., Ltd.), etc. Alkylsilane-treated fine zinc oxide particles are exemplified by MZX-304OTS, MZX-508OTS (Teika Corporation), FINEX-30-OTS, FINEX-50-OTS (Sakai Chemical Industry Co., Ltd.), etc.

Producing methods for obtaining the hydrophobized inorganic powder referred in the present invention are not specifically limited, thus the hydrophobized inorganic powder may be prepared by mixing organic surface treatment agents with inorganic powder. Mixing methods include dry method, wet method and the like, but not limited thereto, thus a mixer capable of uniform treatment may be used. The mixer is exemplified by for example, Henschel mixers, ribbon blenders, kneaders, extruders, disper mixers, homo mixers, bead mills, etc. After mixing, powder may be obtained by drying using a hot air dryer, spray dryer, flash jet dryer, conical dryer, etc.

The hydrophobized inorganic powder referred to in the present invention is inorganic powder having hydrophobic property. Evaluation method for hydrophobicity is such that charging 100cc of purified water to a 200cc glass beaker, dropping 0.2g of powder on a spatula from a height of 2cm above the water surface, stirring 50 rounds with the spatula at a speed of 2 rounds per second, and then allowing to stand for 30 seconds. Preferable powders are those, when observed in the water, powder particles do not migrate into water phase, but float. An effect of the present invention cannot be exerted by powders that powder particles migrate into water layer even at a small amount and powders that have not been hydrophobized. There are some powders that partially migrate into aqueous phase even though they have been subjected to surface treatment with organic compounds. They were experimented by Comparative Example 36 of the present invention. Powders that have not been hydrophobized with organic compounds (untreated powders) exhibit that almost powder particles migrate into aqueous phase under experiments in the present application.

Component ratio of the aqueous dispersion of the present invention is explained below. The aqueous dispersion of the present invention preferably contains hydrophobized inorganic powder as component (C) at 40wt% or more. The amount of hydrophobized inorganic powder less than 40wt% provides worsening in formulation design for cosmetics and suitability in use in manufacture. In an aspect of suitability in use, more preferable is an aqueous dispersion containing 50wt% or more of hydrophobized inorganic powder. The suitability in use referred in the present invention means the degree of freedom [flexibility] upon adding powders to cosmetic components. That is, it is because main component is the aqueous dispersion, but not aqueous phase components at a timing that the aqueous dispersion of the present invention is added to cosmetic components. The hydrophobized inorganic powder as the main component of the aqueous dispersion has a purpose of exerting their functions in cosmetics and of omitting dispersion process in which powder dust is generated during manufacturing process of cosmetics, so as to simplify the manufacturing process to reduce costs. The aqueous dispersion of the present invention is an intermediate material capable of being added as a material during the manufacturing process of cosmetics. The higher amount (content) [adoptable amount] of the hydrophobized inorganic powder in the aqueous dispersion realizes the higher degree of freedom [flexibility] in its formulation, resulting in improvement in its suitability in use.

Component ratio of polyoxyethylene (5 to 15) isostearyl ether as component (A) and hydrophobized inorganic powder as component (C) is component (A)/component (C) = 0.5 to 30.0/100.0 (wt%). The component (A) is a component for making the hydrophobized inorganic powder to be dispersed in water and to be stabilized. Too small component ratio relatively to that of the hydrophobized inorganic powder results in insufficient dispersiveness. Too large component ratio results in poor dispersion stability and increased costs, and provides sticky texture (or feeling) upon contained in cosmetics, thus undesirable in terms of sensory property.

Producing method of the aqueous dispersion of the present invention may be selected from the following optional cases: polyoxyethylene (5 to 15) isostearyl ether as the component (A) is dissolved in or mixed with water as the component (B), and then the hydrophobized inorganic powder as component (C) is charged thereto; and polyoxyethylene (5 to 15) isostearyl ether as the component (A) is made to contact or mixed with hydrophobized inorganic powder as the component (C), and then the mixture is charged to water as the component (B). The methods of mixing or contacting the component (A) with the component (C), or mixing/dispersion methods of 3 components of the component (A), the component (B) and the component (C) are not specifically limited, thus any known mixing/dispersing machines may be used. Usable mixers include, for example, propeller stirrer, disperse mixer, homo mixer, high-pressure homo mixer, kneader, Henschel mixer, V-type mixer, roll mill, bead mill, extruder, etc.

The aqueous dispersion of the present invention may be applied to the following cosmetics. The cosmetics referred to in the present invention are exemplified by emollient cream, cold cream, whitening cream, emulsion, lotion, essence, pack, carmine lotion, liquid facial cleanser, facial cleansing foam, facial cleansing cream, facial cleansing powder, makeup cleanser, body gloss, sunscreen, sunscreen cream/lotion, etc., as skin care cosmetics, makeup bases, powder foundations, liquid foundations, oil-based foundations, stick foundations, presto powders, face powders, white powders, lipsticks, lipstick overcoats, lip glosses, concealer, cheek rouge, eye shadow, eyebrow, eyeliner, mascara, water-based nail enamel, oil-based nail enamel, emulsified nail enamel, enamel top coat, enamel base coat, etc., as make-up cosmetics; hair gloss, hair cream, hair shampoo, hair rinse, hair color, hair brushing agent, etc., as hair cosmetics; cream, lotion, powder, spray-type deodorant products, etc., as antiperspirant cosmetics; and others, emulsion, soap, bath salts, perfumes, etc. as the other products.

### [Examples]

The present invention is explained in detail while referencing to Examples and Comparative examples.

### (Examples 1 to 52 and Comparative examples 1 to 52)

Prepared were aqueous dispersions of the present invention of components indicated in Tables 1, 5, 9 below. Tables 2, 6, 10 indicate aqueous dispersions having components as Comparative examples. All units [unit of all numerical values] indicated in the tables are wt%. All aqueous dispersions were prepared at 2kg scale. Operations were as follows. First, polyoxyethylene (5-15) isostearyl ether was added (charged) to ion-exchanged water, then heated to 50° C so as to be mixed/dissolved, and then stirred with a disper mixer for 1 minute. Hydrophobized inorganic powder was gradually added to the mixture under stirring with a disper mixer so as to be dispersed for 20 minutes at a peripheral speed of 5 m/s. The dispersion was dispersed in a sand grinder with a vessel capacity of 300 cc. Dispersion conditions are as follows: beads are zirconia beads 1.0 mmcp, bead filling ratio 85%, disk peripheral speed 6 m/s, feed rate 30 cc/min, and 4 round passes. The total residence time per one round pass in the mill was 5 minutes. As for Example 27, Example 29, Comparative Example 27, Comparative Example 29, the sand grinder dispersion was performed in 3 round passes, not in 4 round passes, and for Example 34 and Comparative Example 34, the sand grinder dispersion was performed in 2 round passes. Furthermore, in Example 19, Example 26, Example 33, Example 37, Example 26, and Example 33, the aqueous dispersions of the invention were prepared by only dispersing with a dispersion mixer for 20 minutes without the sand grinder dispersion. The comparative examples were subjected to dispersion tests using surfactants described in Examples of JP2018-24881A. Furthermore, in order to observe dispersiveness in water and long-term storage dispersion stability of weakly hydrophobized inorganic powder and non-hydrophobized inorganic powder (untreated powders), Example 38, Comparative Examples 36, 37, and 38 are designed. 100 cc of these prepared aqueous dispersions ware taken in test containers (100 cc wide-mouth polypropylene containers: AS ONE I-BOY) so as to be evaluation samples for immediate tests and over-time storage evaluation tests. Herein, the aqueous dispersions of contents of Comparative Examples did not become partially fluid aqueous dispersions, there were cases that their viscosity was so increased during dispersion process that some of them could not be transferred as liquid and some of them became solid. In such cases, the products were designated as "X" as a meaning of impossible to product. Factors causing such increased viscosity or solidification are thought to include inappropriate selection of surfactant relative to hydrophobized inorganic powder and shortage of the amount of surfactant, and both factors are thought to provide insufficient wettability of the hydrophobized inorganic powder in water and poor adsorption of the surfactant on the powder particle surface.

Methods for storage stability tests of the aqueous dispersion of the present invention are described below. PP test container containing the aqueous dispersion of the present invention is stored in a temperature-humidity test chamber described below, while the container is tightly shielded with a lid. A gap between the lid and the container is covered with vinyl tape by one and a half rounds of the gap. Conditions for each storage tests are as follows.

### (Cycle test: -20°C to 40°C/2 weeks)

A test container containing the aqueous dispersion is stored in a test chamber. The test container is set to be subjected to the following cycles: temperature rising from -20°C to 40°C over 12 hours and temperature falling from 40°C to -20°C over 12 hours (i.e., repeated temperature rising and falling of 5°C/1 hour). After 2 weeks, the test container is taken out.

### (Cycle test: 5°C to 60°C/2 weeks)

A test container containing the aqueous dispersion is stored in a test chamber. The test container is set to be subjected to the following cycles: temperature rising from 5°C to 60°C over 12 hours and temperature falling from 60°C to 5°C over 12 hours (i.e., repeated temperature rising and falling of 4.583°C / 1 hour). After 2 weeks, the test container is taken out.

### (Acceleration test: 40 ± 2°C/75%RH± 5%RH/6 months)

A test container containing the aqueous dispersion is stored in a test chamber. The test container is set to have an internal temperature of 40±2°C and a humidity of 75%±5%. After 6 months, the test container is taken out. The test container is not completely airtight and thought that humidity affects thereon, thus the humidity is set at 75%±5%.

### (RT storage test/ 1 year)

RT means room temperature, and a container is stored in a laboratory cabinet for one year. A laboratory is controlled to have conditions throughout the year of temperature:15-25°C and humidity: 35-80%. After one year, the container is taken out from the laboratory cabinet. It is confirmed that whether a lid becomes loose for the test container taken out after completion of the test. There was no test container having a loose lid.

The aqueous dispersion of the present invention was evaluated as follows.

### (Appearance evaluation of the aqueous dispersion)

Aqueous dispersions of immediately after preparation and after completion of storage stability test are placed in a thermostatic bath at 25°C for 24 hours. Then, supernatant is observed with eyes and bottom of the container is observed using 180 mm microspatula to evaluate precipitation state. If the aqueous dispersion has a poor stability. a supernatant layer or precipitation layer is observed. The supernatant layer indicates states in which dispersion layer of powder particles is not uniform, and a thin dispersion layer of powder particles is observed in upper layer, or a clear aqueous phase is observed. The precipitation layer also indicates states in which dispersion layer of the powder particles is not uniform and a layer of precipitated particles is observed. Since the powder particles has a specific gravity lager than that of the aqueous phase as dispersion medium, the powder particles are precipitated over time. Such precipitation state included a soft cake state and a hard cake state. The former state can be easily re-dispersed by stirring with a spatula and the like to recover a uniform dispersion phase, while the latter state is difficult to be re-dispersed even using a spatula or mechanical stirring. In the storage stability test, it is particularly preferable that realizes a state of no-supernatant layer and no-precipitation layer. It is allowable that even though supernatant layer or soft cake layer is provided, if they may be easily redispersed by stirring with a spatula or the like. As for evaluations, the aqueous dispersions are evaluated as CΔ if supernatant is observed, and evaluated as Co if no supernatant is observed. The aqueous dispersions are evaluated as So if no precipitation is observed, evaluated as SΔ if soft cake is observed, and evaluated as SX if hard cake is observed. Some aqueous dispersions are solidified after each storage stability test, and evaluated as Solidified X. Solidification [solid state] refers to a state in which liquid material hardens into a clay-like state that cannot recover liquid state by stirring or other physical stimulation. It is probably due to absorption of liquid by powder or volatilization of liquid.

### (Viscosity of aqueous dispersion)

Viscosity of the aqueous dispersion is evaluated as follows. 100cc PP test container containing the aqueous dispersion is placed in a thermostatic bath at 25°C for 24 hours. After that, the aqueous dispersion is manually stirred for 30 seconds with the microspatula so as to prepare a viscosity measurement sample. Bismetron VDA viscometer manufactured by Shibaura Semtech Co. is used as a viscometer, and No. 3 rotor is used. Rotational speed of the rotor is set at 30 rpm and 60 rpm for 60 seconds for both speeds. As measured value, for example, a case in which the viscosity after 60 seconds at 30 rpm is 300 mPa-s and after 60 seconds at 60 rpm was 600 mPa-s is labeled as 300/600. No. 3 rotor has a measurement capacity range of 400 to 4000 mPa-s at 30 rpm and 200 to 2000 mPa-s at 60 rpm. If the aqueous dispersion has a higher viscosity that is impossible to be measured at the above rotor speeds, rotor speed is reduced to 12 rpm (measurement capacity range: 1000-10000 mPa-s) or 6 rpm (measurement capacity range: 2000-20000 mPa-s). Aqueous dispersions containing hydrophobized powders are non-Newtonian fluids, thus their viscosity depends on their shear rate. Therefore, the viscosity is measured under two levels of rotor speeds. In such case, measurement at a lower speed is performed in advance. Herein, a dispersion that becomes a hard-cake state cannot be redispersed by manual stirring with a spatula, thus its viscosity measurement is labeled as impossible measurement X.

### (Gloss test)

The aqueous dispersion of the present invention is taken onto a TAC (triacetyl cellulose) film (Lonza TAC100, 100 µm thickness: PANAC Corporation), spread to be coated using #6 bar coater, and dried in a constant temperature oven at 80°C for 1 hour so as to prepare an evaluation sample. As a gloss measurement machine, a spectrocolorimeter manufactured by a Nippon Denshoku Kogyo is used under the following conditions. Light source is C light source, measurement method is reflection method, measurement angles are incident angle: - 45°, measurement angle: +45°. As a standard plate, a white plate for color measurement is used. Herein, in the measurement, five different points on the TAC film are subjected to the measurement, and an average value is obtained as a gloss value. The higher gloss value indicates the better dispersibility of the hydrophobized inorganic powder particles in the aqueous dispersion. Poor storage stability provides aggregation of the particles, results in decreased gloss value. If the gloss value after the storage stability test is equivalent to that immediately after manufacture, the stability is excellent.

### (UV shielding test)

With respect to aqueous dispersions containing non-nano titanium dioxide and non-nano zinc oxide and aqueous dispersions containing fine titanium dioxide and fine zinc oxide, the TAC films prepared in the gloss test are used for measuring SPF values with in-vitro SPF value tester. In measurements, Lab sphere UV-2000S (Sanyo Trading Co., Ltd.) is used, uncoated TAC is used as a blank, and an average value of 5 samples (n is 5) is determined as SPF value. Evaluation results are indicated in Tables 3 and 4, etc.

Tables 3, 7 and 11 indicate evaluation results of the aqueous dispersion as the Examples, and tables 4, 8 and 12 indicate evaluation results of the aqueous dispersion as Comparative examples (C-Ex.).

In addition, table 13 indicates primary particle sizes of raw material before hydrophobization of the hydrophobized inorganic powder used in the present invention and hydrophobicity of the hydrophobized inorganic powder. As described above, the primary particle sizes are those published by material manufacturers. Hydrophobicity evaluation method for the hydrophobized inorganic powder is such that: charging 100cc of purified water to a 200cc glass beaker, dropping 0.2g of powder on a spatula from a height of 2cm above the water surface, stirring 50 rounds with the spatula at a speed of 2 rounds per second, and then allowing to stand for 30 seconds. A powder is labeled as ∘ if floating powder particles, but not migrating to water layer are observed, and labeled as X if migrating powder particles to water layer are observed even at a small amount.

The aqueous dispersion of the present invention is measured for pH and secondary particle size after 40±2°C/75%RH±5%RH/6 months. Results are shown in Table 14. pH is measured with portable pH meter (Toa DKK Corporation), calibration is performed with pH standard solutions (pH 4.01, pH 6.86, and pH 9.18), pH is read after 30 seconds of insertion of a pH electrode into the aqueous dispersion solution. Secondary particle size is measured using a laser diffraction scattering particle size analyzer (Malvern: Mastersizer 3000). Purified water is used as dilution solvent. Refractive indices of the hydrophobized inorganic powder, which are input for measurement of particle size, are 2.75 for titanium dioxide, 2.10 for yellow iron oxide, 3.01 for red iron oxide, 2.42 for black iron oxide, and 2.00 for zinc oxide. Each aqueous dispersion is portioned into an amount to be a measurable concentration in advance, diluted with purified water, and charged into a circulation cell without any dispersion operation such as ultrasonic waves, etc. With respect to the secondary particle size, D5, D50, and D95 are determined as volume particle size. Herein, comparative examples are not subjected to particle size distribution measurement, since they have no sample that is stable after acceleration test.

Some of the aqueous dispersions of the present invention, which have an excellent long-term storage dispersion stability, is subjected to measurement of zeta potential. Measurement method is as follows. Used machine for zeta potential is ELSZ-1000 Zeta Potential and Particle Size Measurement System (Otsuka Electronics Co., Ltd.). Principle of the measurement is electrophoretic light scattering method, also known as laser Doppler method. When incident light is applied to a system in which electrically charged particles are dispersed in water, the frequency of the light is shifted due to the Doppler effect, by which light reflected or scattered by the particles changes in proportion to the speed of the particles. Since the amount of shift is proportional to the electrical charge of the particles, thus zeta potential may be determined.

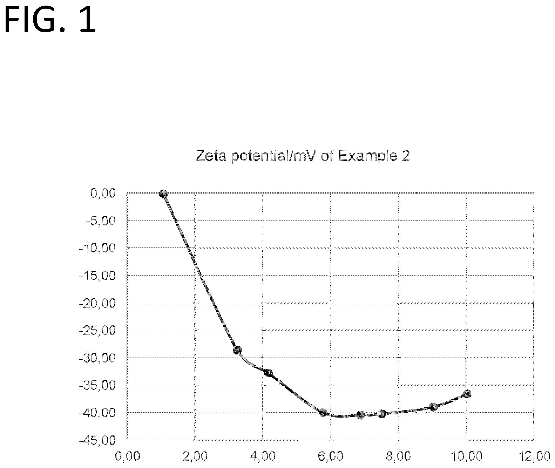

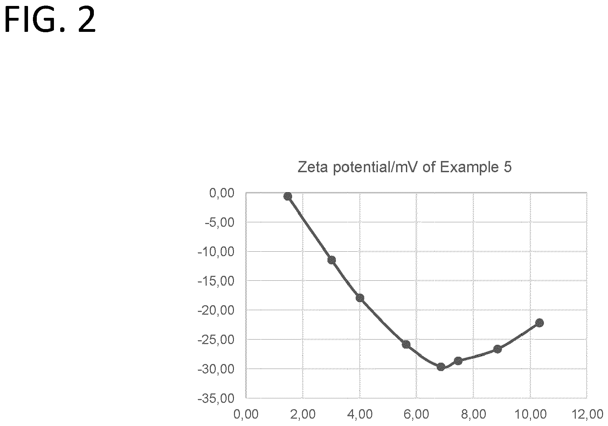

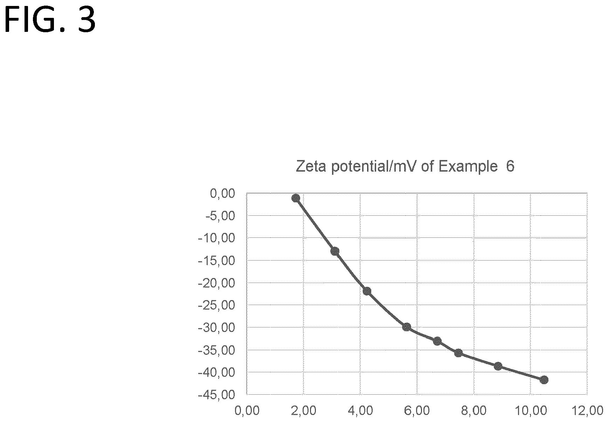

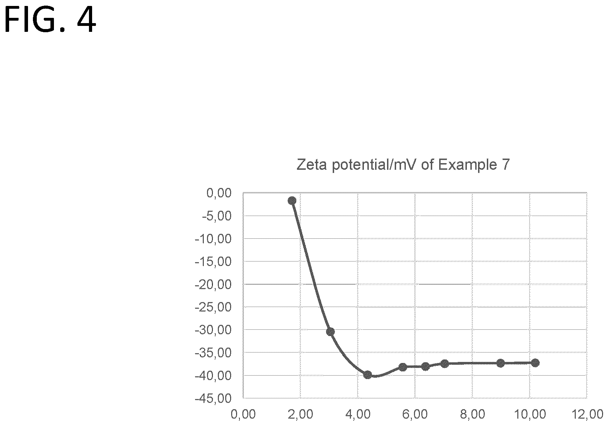

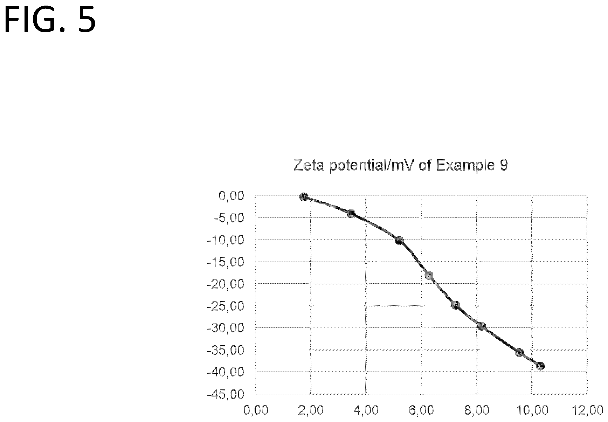

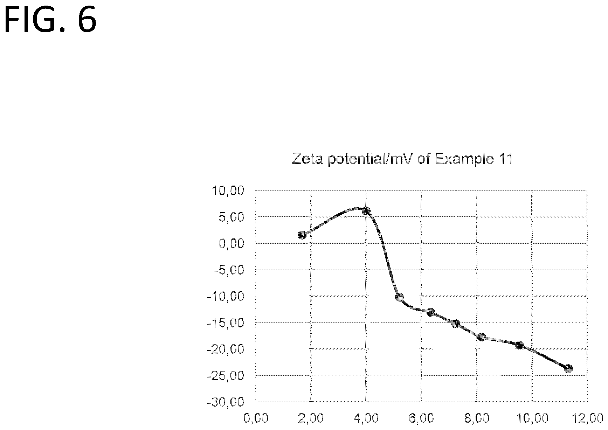

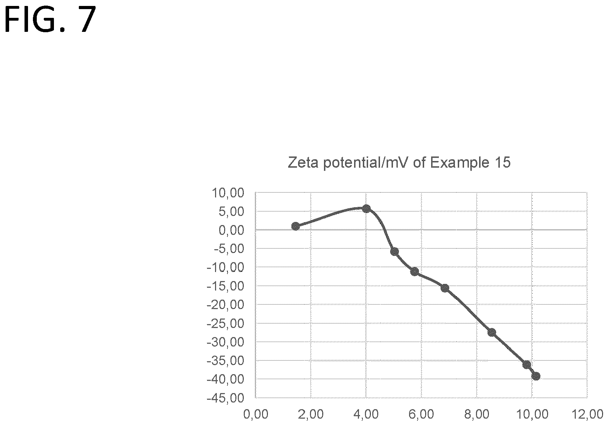

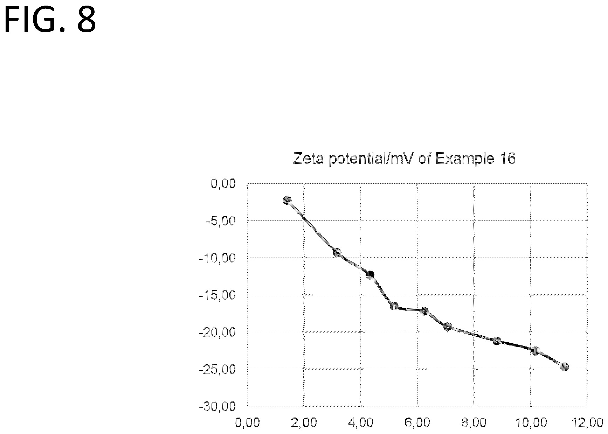

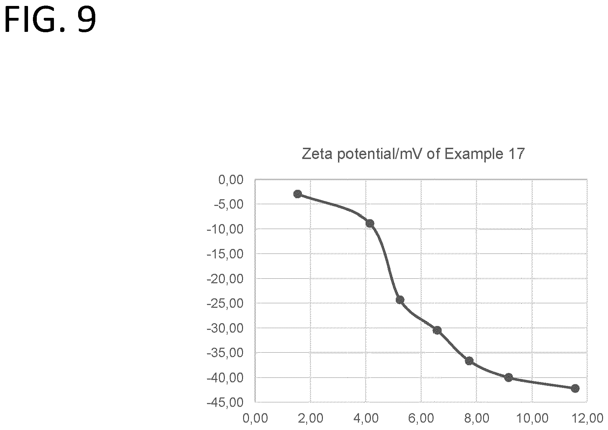

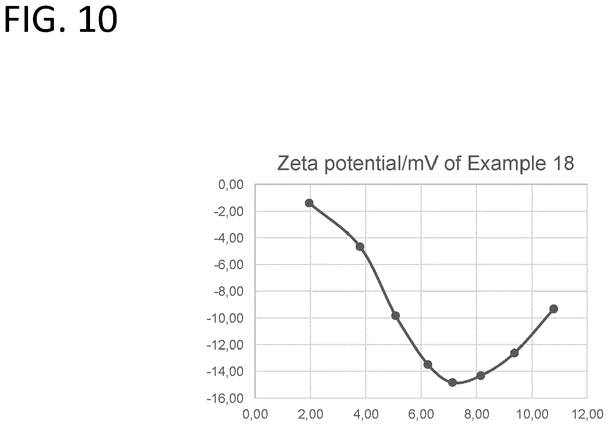

Zeta potential measurement procedure is as follows. First, the aqueous dispersion is diluted with ion exchanged water to have a concentration of hydrophobized inorganic powder particles of 0.005 to 0.1wt%. If the powder particles are poorly dispersed, dispersion process using an ultrasonic dispersion machine or a magnetic stirrer is performed, if necessary, to prepare measurement solution. A reason for the powder particle concentration having a usable range is that the diluted dispersion solution has a different light transmittance due to primary particle size of inorganic powder particles, particle size when being dispersed in water, shape and refractive index of the inorganic powder particles. In order to regulate to a transmittance capable of measuring zeta potential, particle concentration may be different from one another. Dispersion solution having a measurable particle concentration is prepared, charged into a measurement cell, and subjected to measurement for zeta potential 25°C. During measurement, a pH titrator automatically controls pH. Manual control is performed as follows. As measurement solution, seven to nine aliquots of pH solution are prepared for each of pH within a range of pH 1 to pH 10. NaOH is used for basic conditions, and HNO₃ is used for acidic conditions. HCl is unpreferable, because when HCl is used for preparation of pH solution, Cl- (negative) ions is attached onto particle surface and provide a negative charge. Figures 1-10 shows measured charts for zeta potential. Herein, horizontal axis in Figures 1-10 indicates pH.

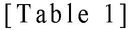

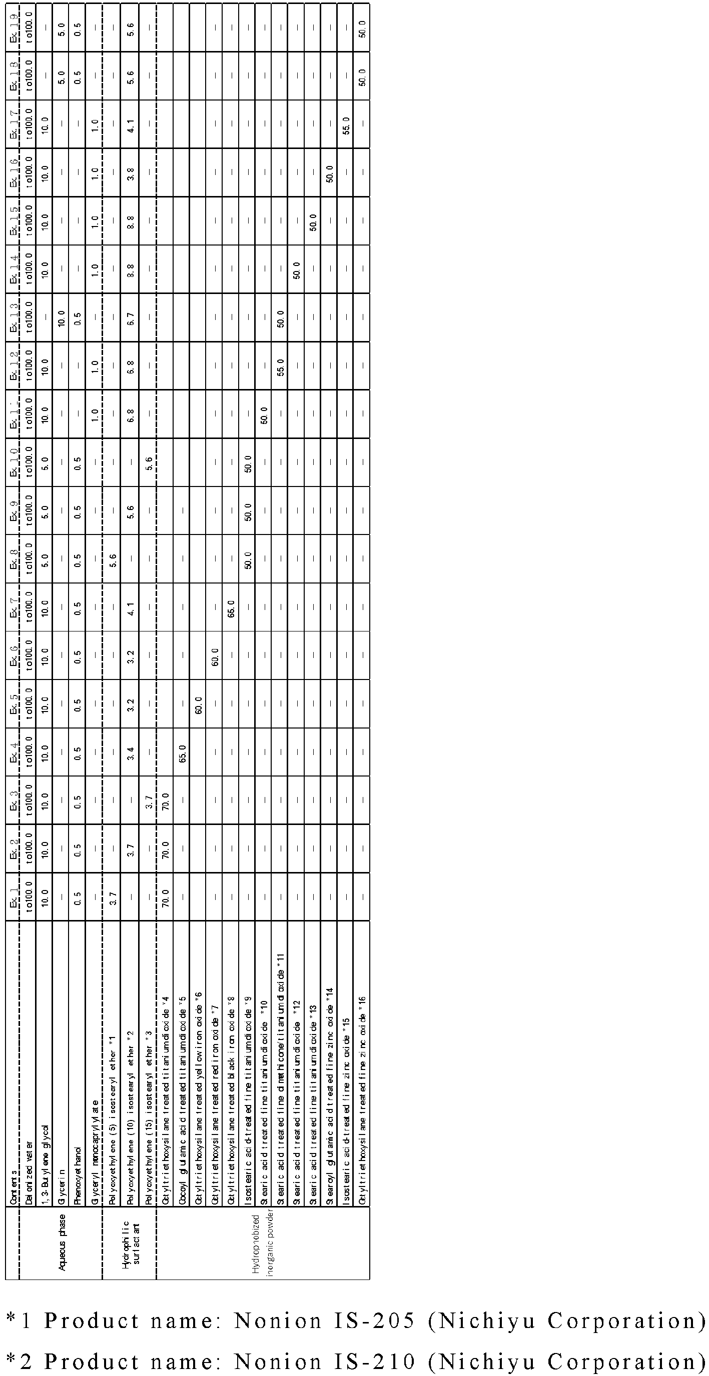

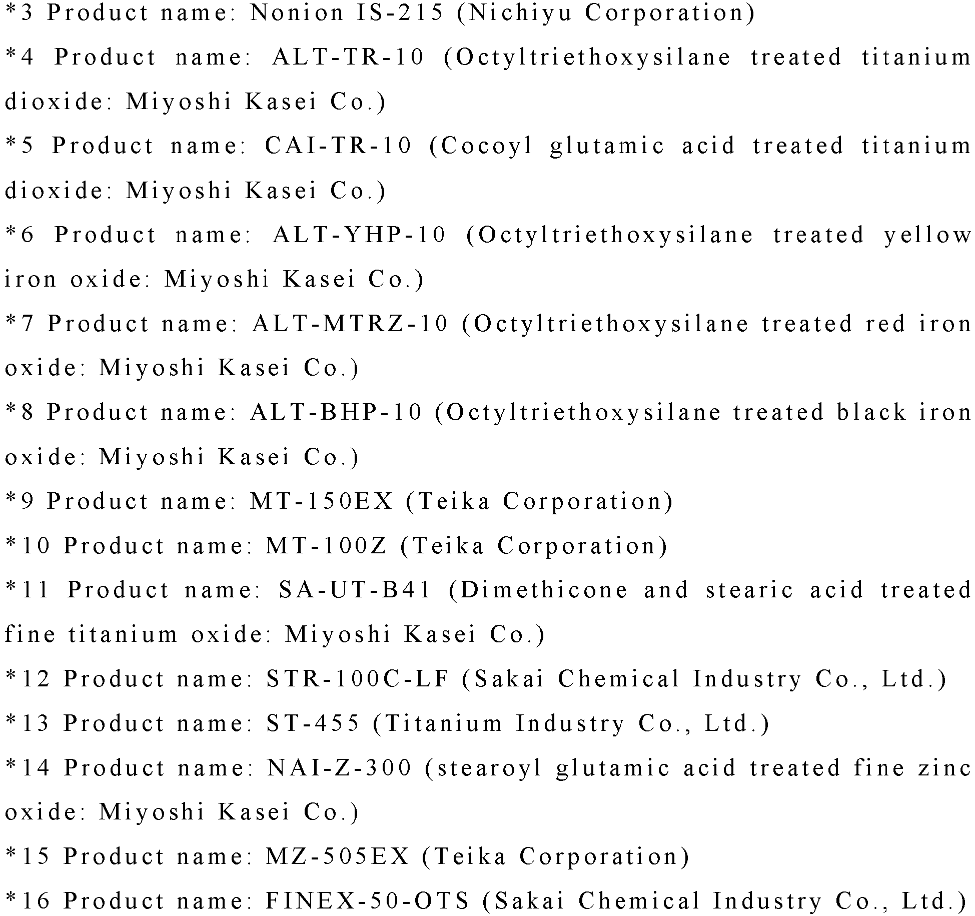

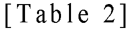

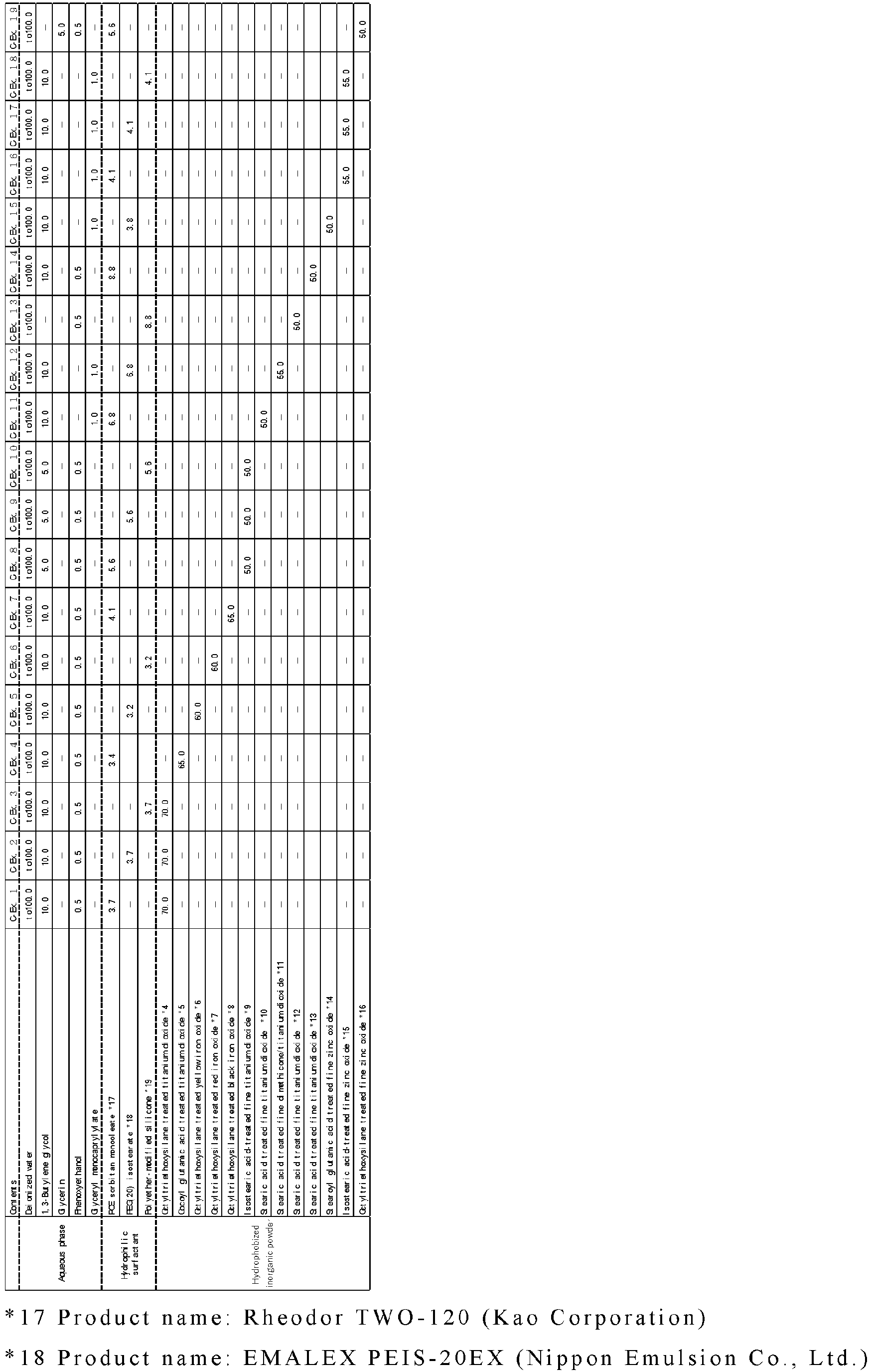

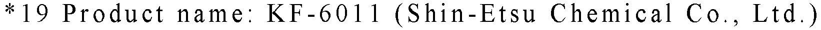

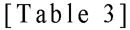

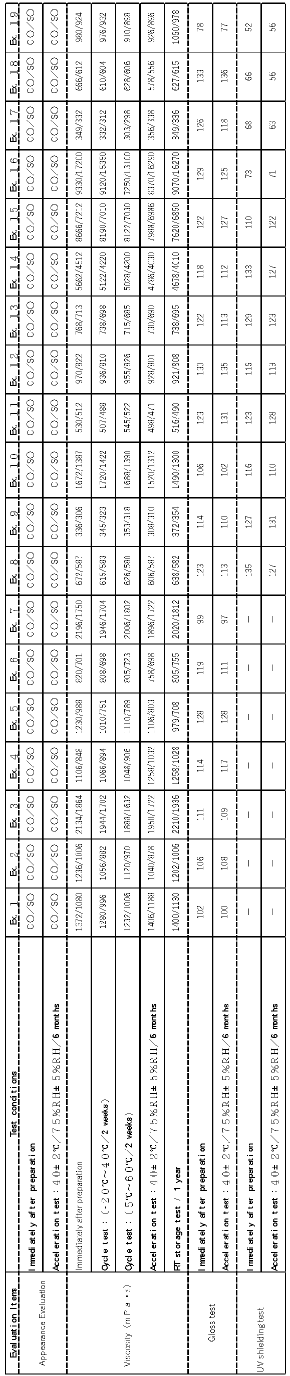

The aqueous dispersion of the present invention has a low viscosity and an excellent long-term storage stability satisfying both acceleration tests and cycle tests.

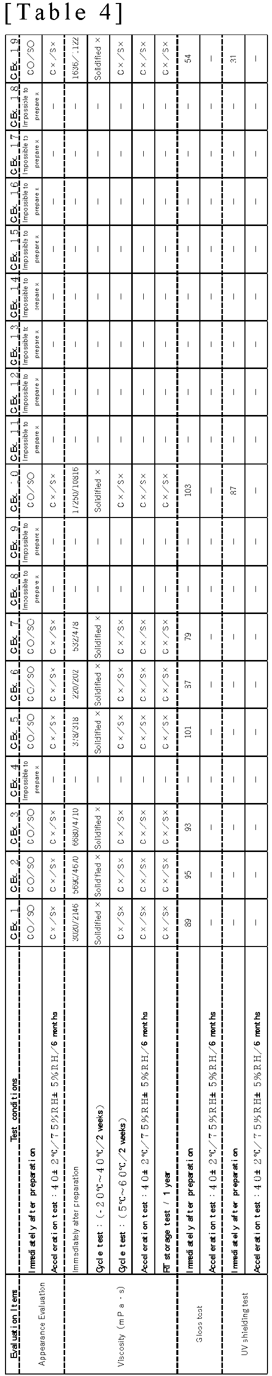

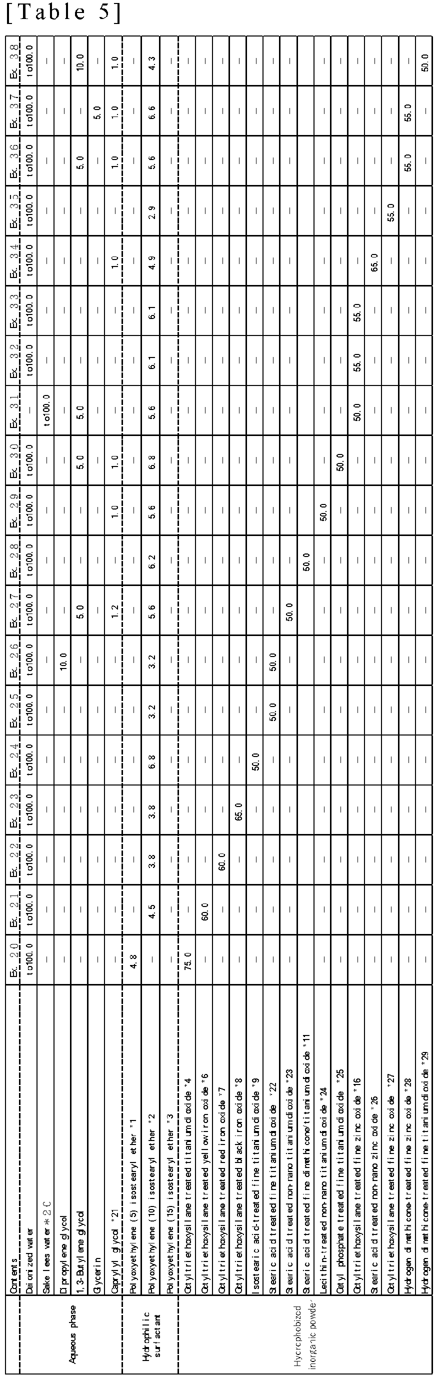

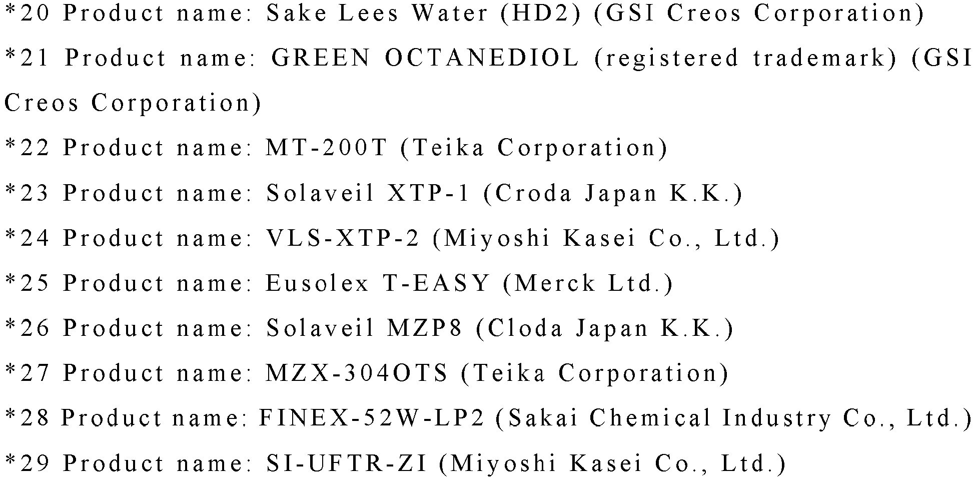

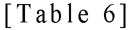

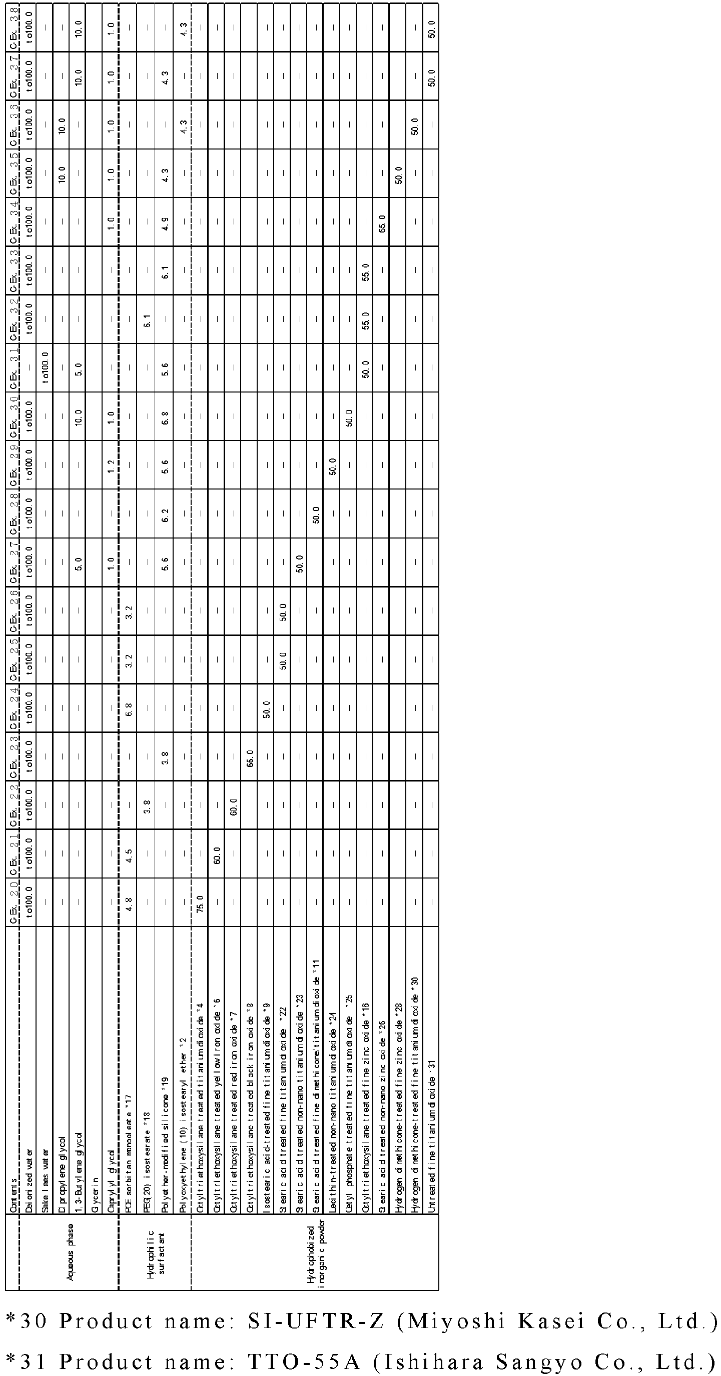

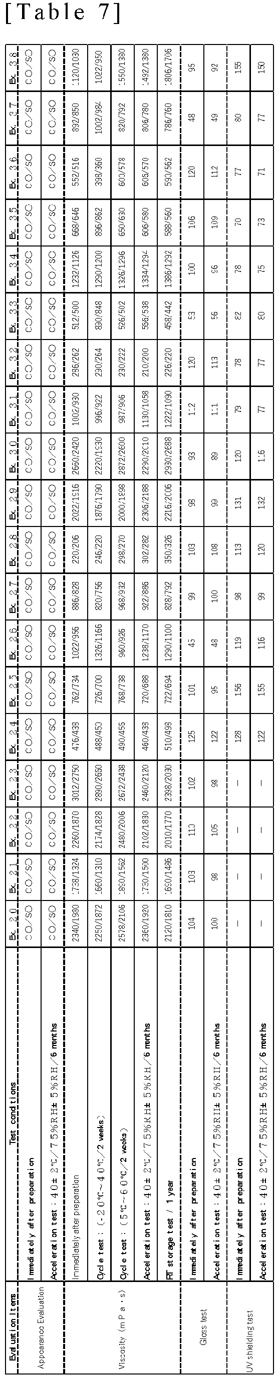

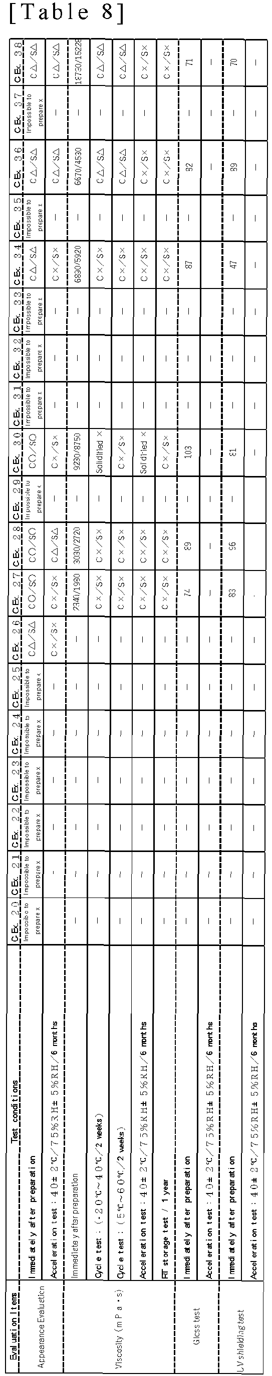

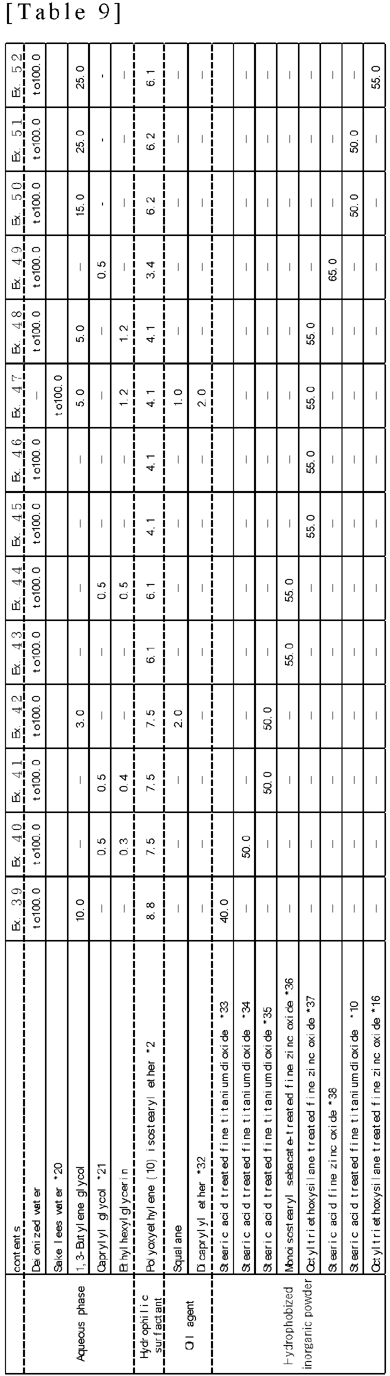

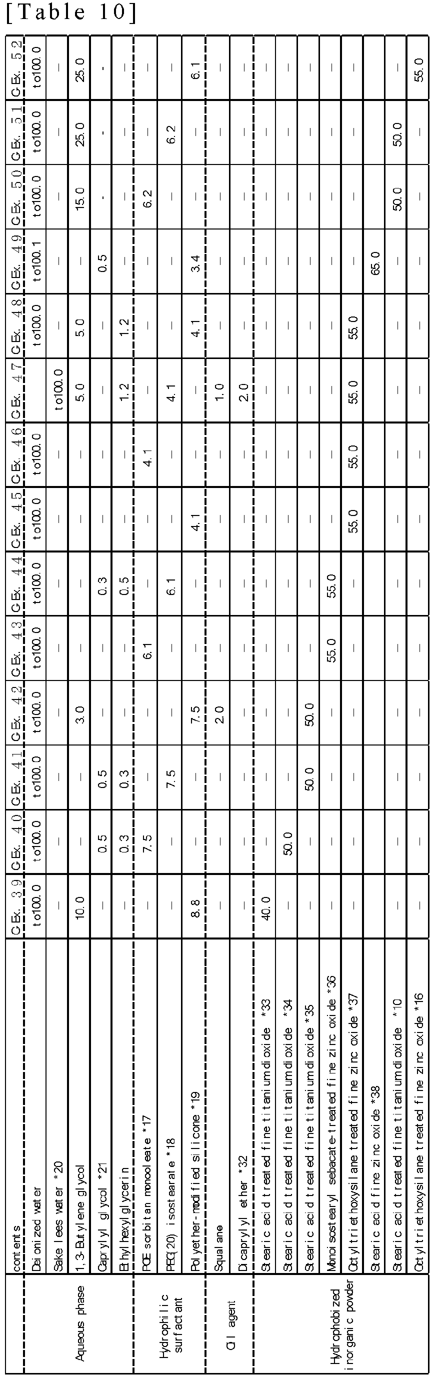

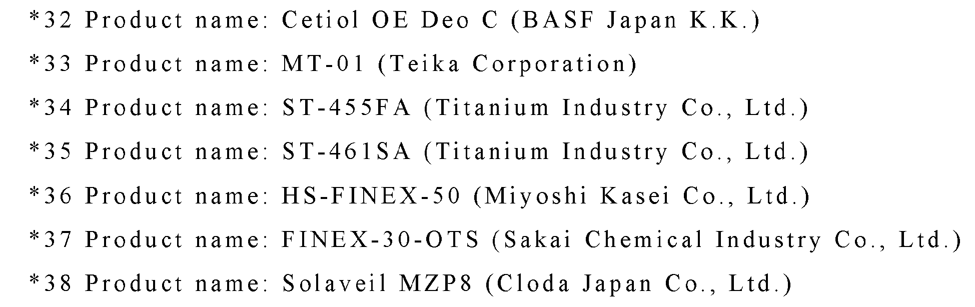

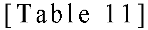

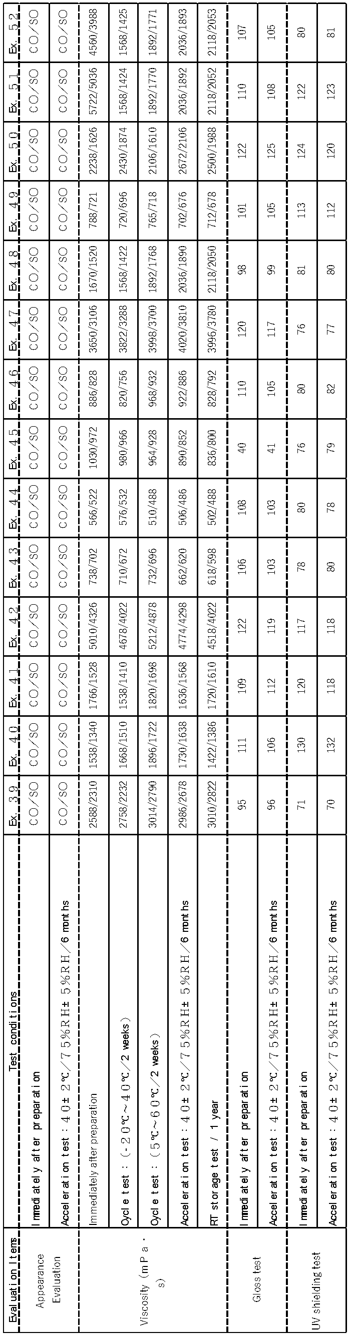

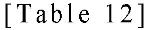

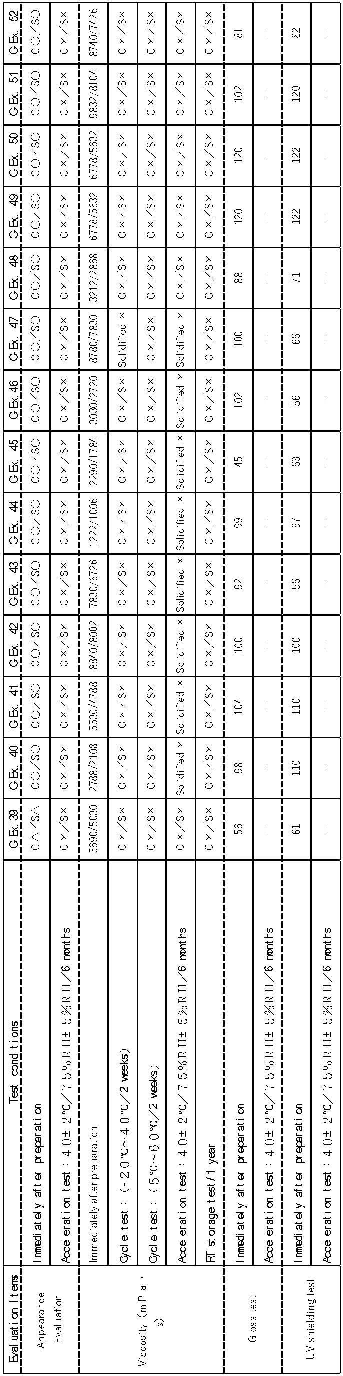

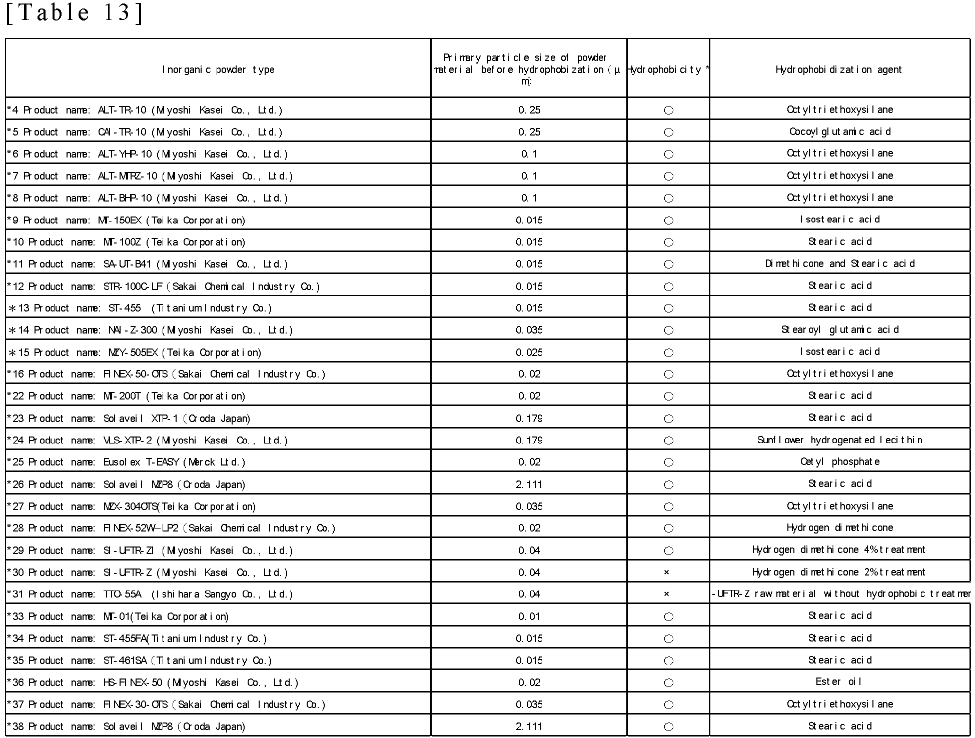

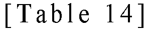

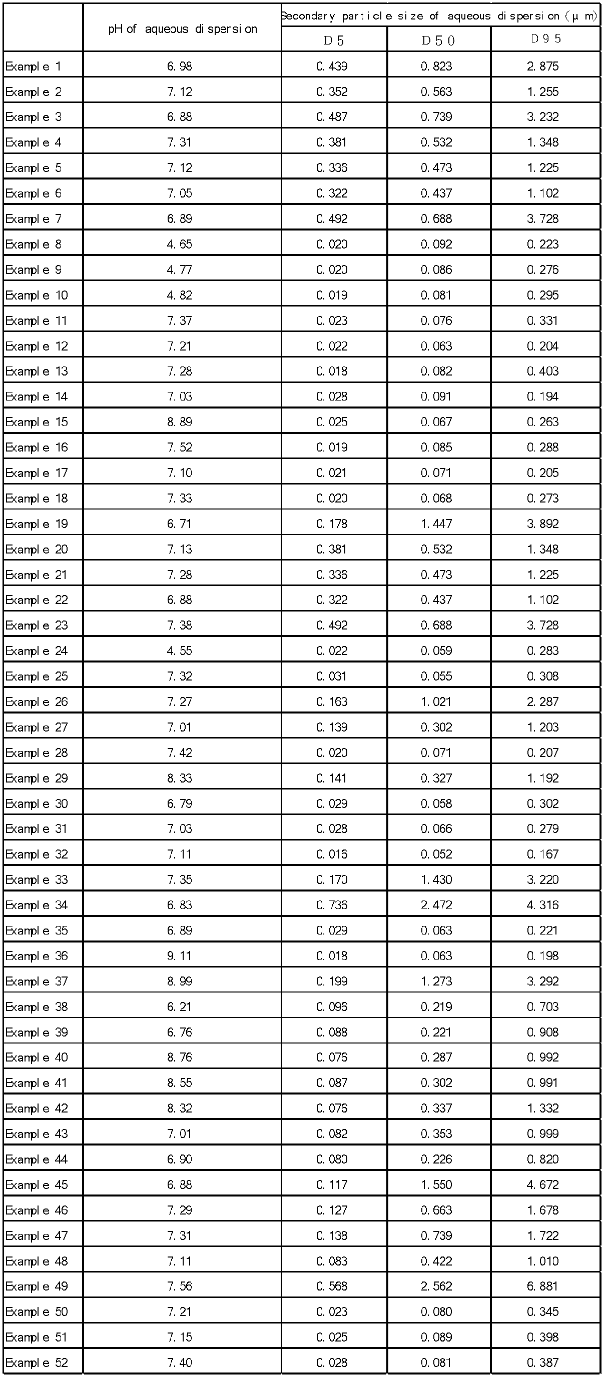

### (Discussion for evaluation results)

All of the aqueous dispersions containing the hydrophobized inorganic powder of the present invention have a liquid state that flows even though they contain the hydrophobized inorganic powder at a high content ratio. The aqueous dispersions have pH between 4 to 5 to around 9, realizing long-term dispersion stability over a wide pH range. The results of zeta potential measurements show that the aqueous dispersions exhibit a negative charge at pH between 4 and 5 to above 10 or more. It is indicated that, within pH range of the aqueous phase used in cosmetics, powder particles are not aggregated and possible to exhibit a performance of the inorganic powder even though these aqueous dispersions are contained.

In addition, in general, when polyhydric alcohols are contained in an aqueous phase, surface tension of the aqueous phase decreases in proportion to contained amount thereof and the hydrophobized powder is easily wetted by the aqueous phase, resulting in easiness of preparation of the aqueous dispersions. However, the aqueous dispersions of the present invention may be prepared only with three components of polyoxyethylene (5-15) isostearyl ether, water, and hydrophobized inorganic powder even in systems in which no polyhydric alcohols are contained, that is, systems in which the surface tension of the aqueous phase is not reduced by polyhydric alcohols, such as Examples (Ex.) 20-25, resulting in a long-term storage stability.

Furthermore, the aqueous dispersions of the present invention may be prepared even under moderated dispersion conditions like as Examples 27 and 29 (three passes with a sand grinder), Example 34 (two passes with a sand grinder), and Examples 26, 33, and 37 (with a dispersion mixer only). In other words, the aqueous dispersions of the present invention may be easily prepared. With respect to secondary particle size of the aqueous dispersions of the present invention after storage stability tests as shown in Table 14, surprisingly, it was found that long-term dispersion stability was achieved even under several tens nm of D50 or within a range of submicrons or more. Logical reason is unknown, why the dispersion stability may be maintained over a long time period even though the inorganic powder particles dispersed in the aqueous phase are much larger than Stokes' sedimentation equation theory. It is thought as a synergistic effect derived from negative charges of both the polyoxyethylene (5-15) isostearyl ether as dispersant used in the present invention and the hydrophobized inorganic powder particles in the aqueous dispersions.

### (Example 53: Preparation of O/W emulsified foundation)

An O/W emulsified foundation of compositions shown in Table 15 was prepared.

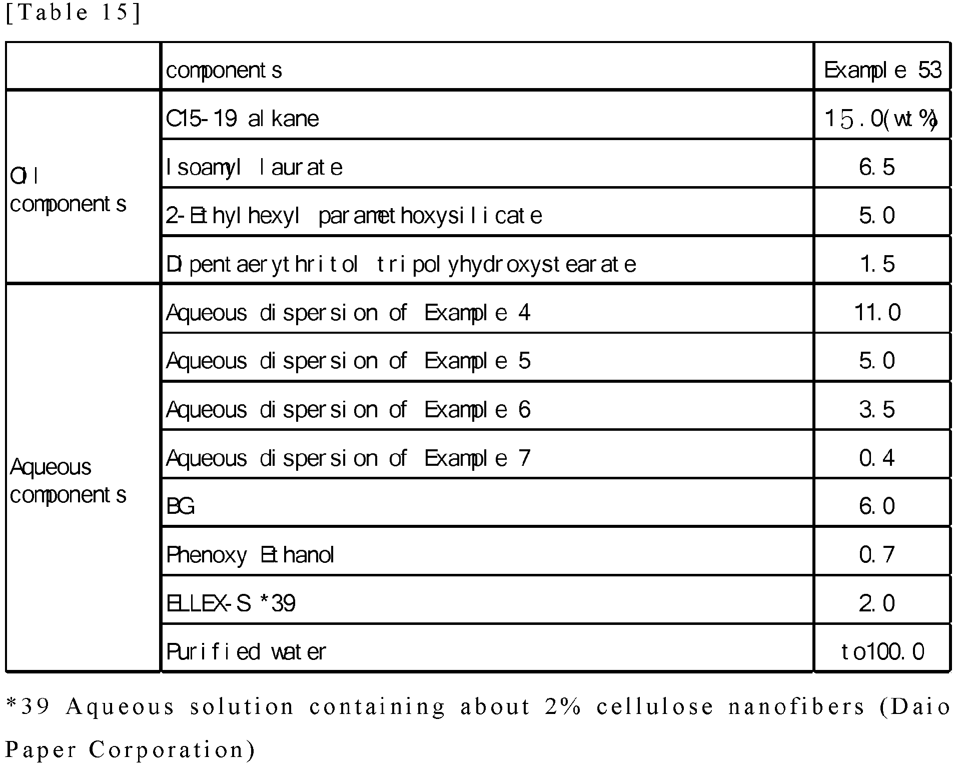

### (Preparation method)

A: Disperse and mix oil phase components well.
B: Disperse and mix aqueous phase components well.
C: Add A to B and emulsify with a homo mixer to obtain an O/W emulsified foundation.

The O/W emulsified foundation of the present invention had a good dispersibility of powders, a good using feeling and coloration, uniform finish, and good stability.

### (Example 54: Preparation of W/O emulsified foundation)

A W/O emulsified foundation of compositions shown in Table 16 was prepared.

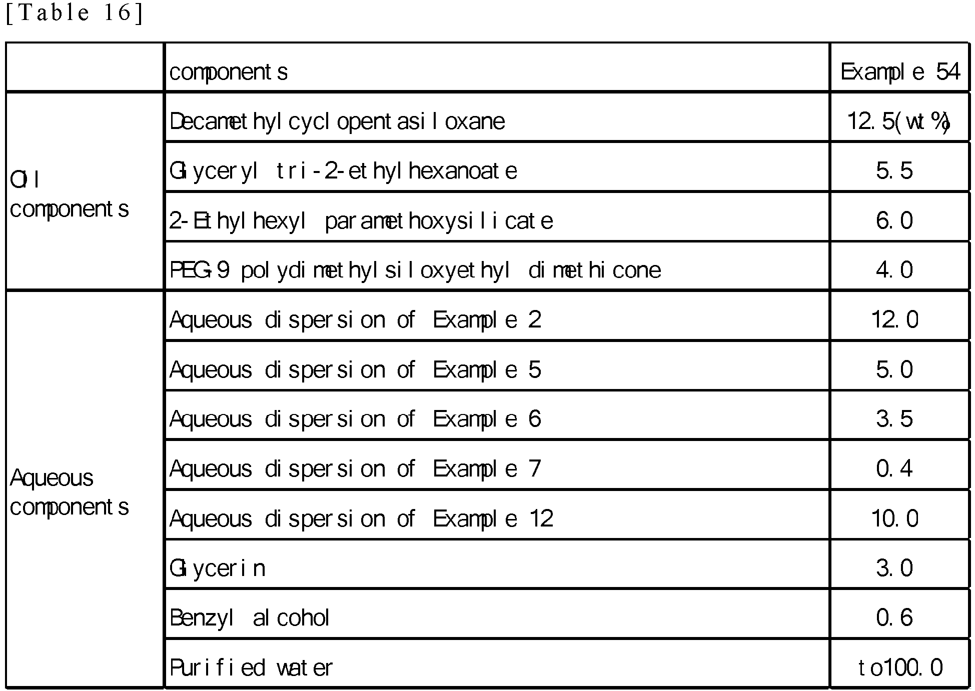

### (Preparation method)

A: Disperse and mix oil phase components well.
B: Disperse and mix aqueous phase components well.
C: Add B to A and emulsify with a homo mixer to obtain a W/O emulsified foundation.

The W/O emulsion foundation of the present invention had a good dispersibility of powders, a good coloration, less stickiness, fresh feel, uniform finish, and good stability.

### (Example 55: Preparation of O/W sunscreen cosmetic)

An O/W sunscreen cosmetic of compositions shown in Table 17 was prepared.

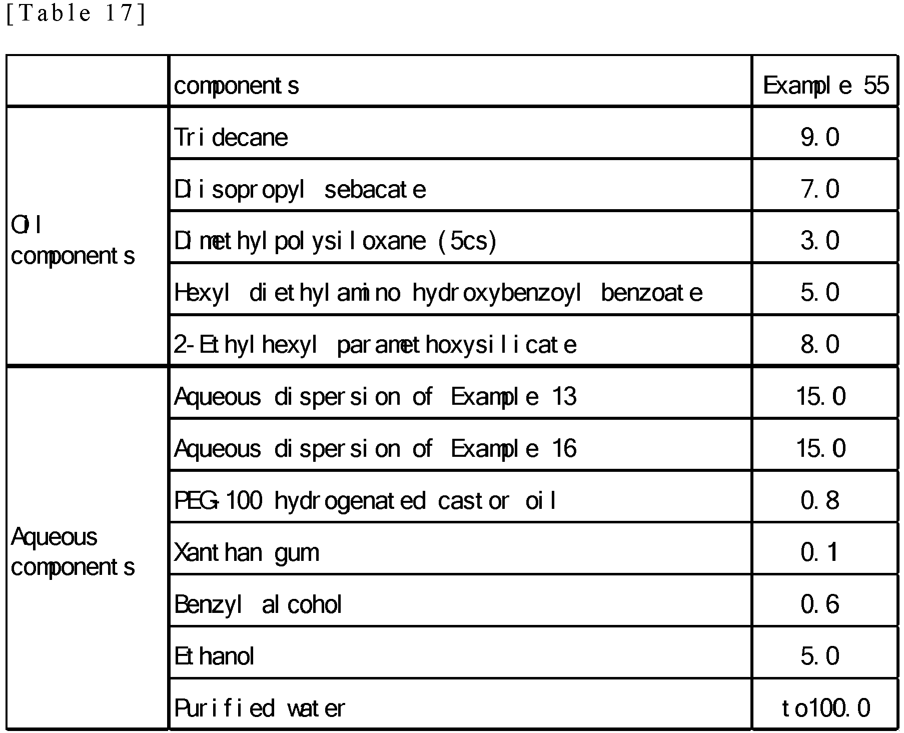

### (Preparations method)

A: Disperse and mix oil phase components well.
B: Disperse and mix aqueous phase components well.
C: Add A to B and emulsify with a homo mixer to obtain an O/W type sunscreen cosmetic.

The aqueous dispersion of Example 13 contains hydrophobized fine titanium dioxide particles, and the aqueous dispersion of Example 16 contains hydrophobized fine zinc oxide particles. Normally, when fine titanium dioxide particles and fine zinc oxide particles are mixed in an aqueous phase, their charges attract one another, resulting in aggregation thereof. However, the aqueous dispersion of the present invention did not result in aggregation in the aqueous phase even when titanium dioxide and zinc oxide were mixed.

The sunscreen cosmetic of the present invention had a good dispersibility of powders, fresh feeling and good stability. In-vitro SPF value was 50 and PA was +++, thus the sunscreen cosmetic had a high UV shielding effect.

### (Example 56: Preparation of lotion-type sunscreen cosmetic)

A lotion-type sunscreen cosmetic of compositions shown in Table 18 was prepared.

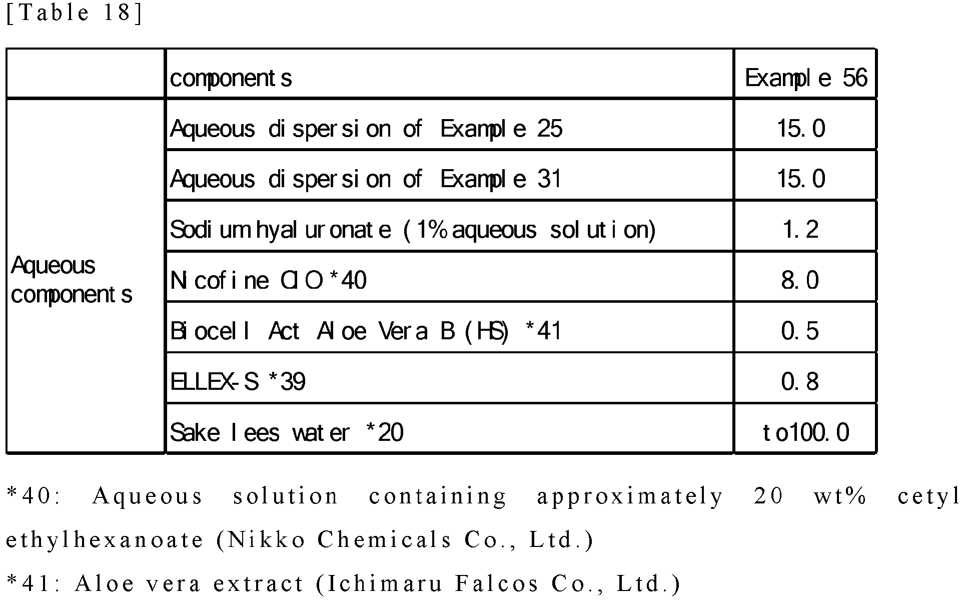

### (Preparation method)

A: Mix aqueous phase components to obtain a lotion-type sunscreen cosmetic.

The lotion-type sunscreen cosmetic of the present invention had a good dispersibility of powder, fresh feeling, smooth texture, but not sticky texture, and good stability. In-vitro SPF value was 38 and PA was ++, thus the lotion-type sunscreen cosmetic had a high UV shielding effect.

### (Example 57: Preparation of W/O sunscreen cosmetic)

A W/O sunscreen cosmetic of compositions shown in Table 19 was prepared.

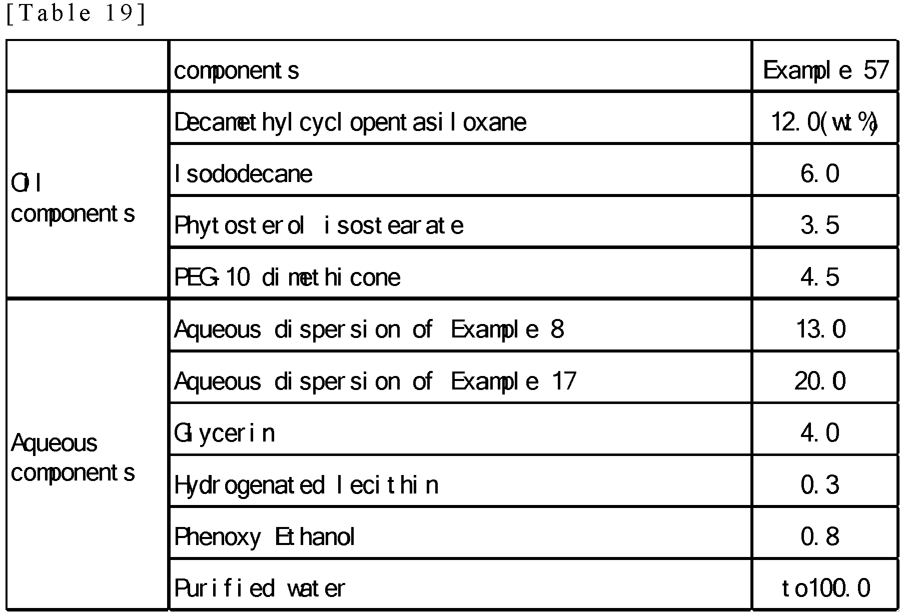

### (Preparation method)

A: Disperse and mix oil phase components well.
B: Disperse and mix aqueous phase components well.
C: Add B to A and emulsify with a homo mixer to obtain W/O-type sunscreen cosmetic.

The sunscreen cosmetic of the present invention had a good dispersibility of powder, good transparency, less stickiness, fresh feeling, and good stability. In-vitro SPF was 48.

### (Example 58: Preparation of aerosol sunscreen cosmetic)

An aerosol sunscreen cosmetic of compositions shown in Table 20 was prepared.

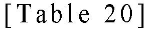

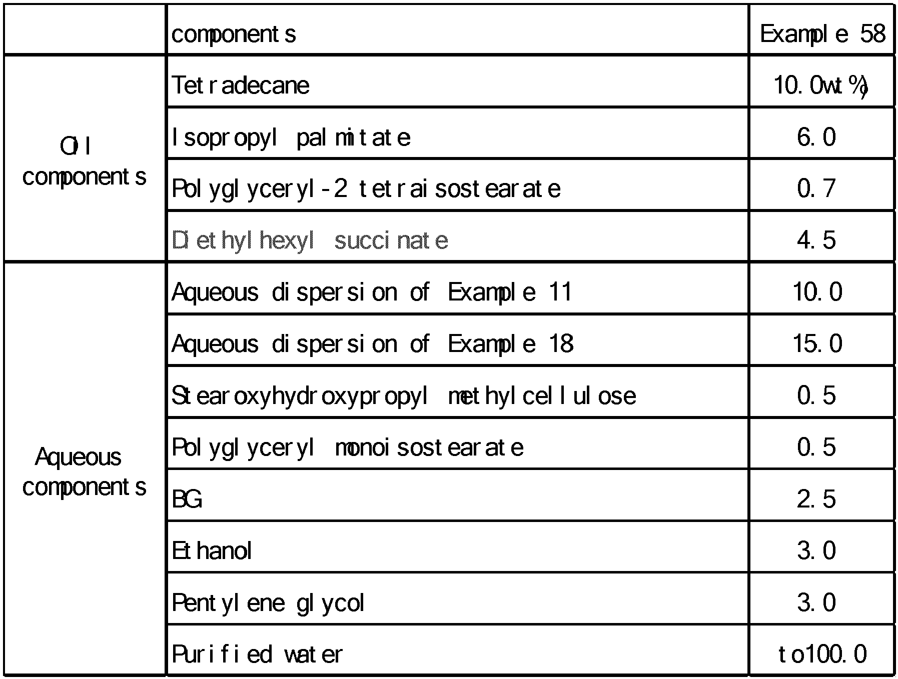

### (Preparation method)

A: Disperse and mix aqueous phase components well.
B: Disperse and mix oil phase components well.
C: Add component B to component A under homo-mixer stirring.
D: Add an injectant to C, and charge into an aluminum pressure-resistant container to obtain an aerosol sunscreen cosmetic.

The aerosol sunscreen cosmetic of the present invention had a good dispersibility of powder, high transparency, very fresh feeling, and good stability. In-vitro SPF value was 39. As shown in the zeta potential graphs of Example 11 and Example 18, a negative charge was exhibited over a wide pH range from weakly acidic to basic, thus no aggregation is provided, and the characteristics of the powder particles are exhibited even though fine titanium dioxide and fine zinc oxide coexist in the aqueous phase.

### (Example 59: Preparation of water-based makeup base)

A water-based makeup base of compositions shown in Table 21 was prepared.

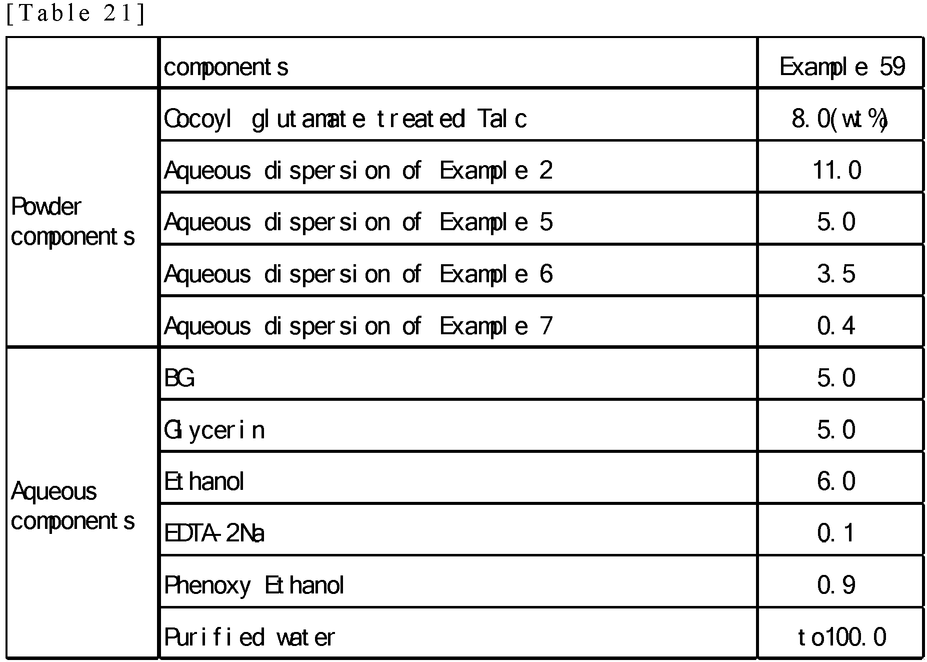

### (Preparation method)

A: Mix powder components well.
B: Add A to mixture of BG (butylene glycol) and glycerin as aqueous phase components and treat with a roller.
C: Add the other aqueous phase components to B and stir it well to obtain a water-based makeup base.

The water-based makeup base of the present invention had a good dispersibility of powder, a fresh feel, and good stability.

### (Example 60: Preparation of water-based facial white)

A water-based facial white of compositions shown in Table 22 was prepared.

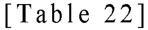

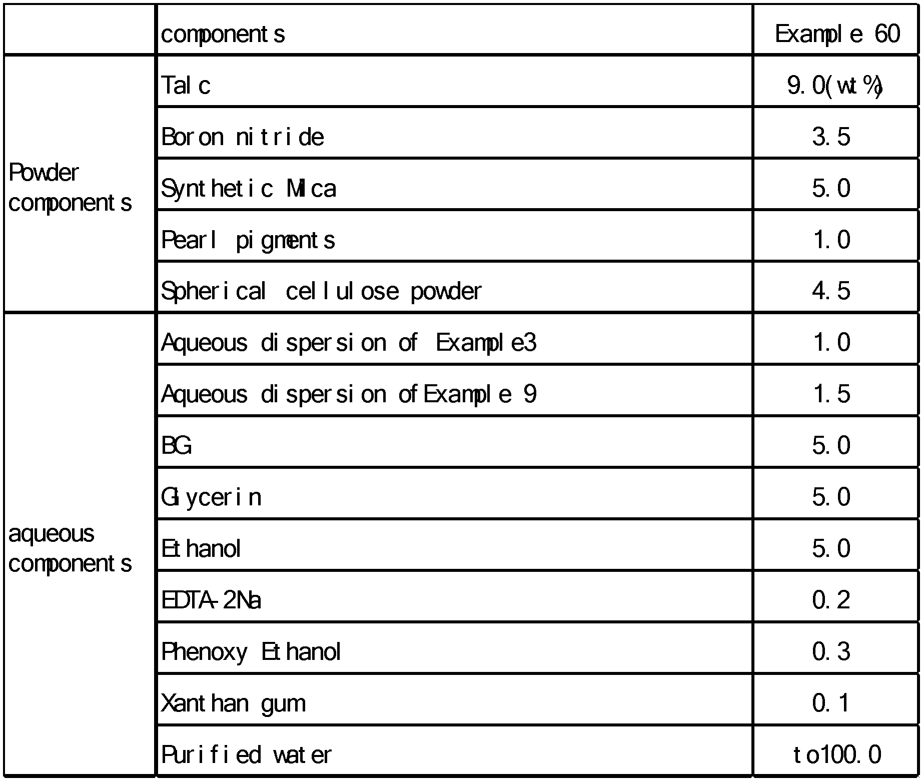

### (Preparation method)

A: Mix powder components well.
B: Mix and dissolve aqueous phase components.
C: Add A to B and stir it well to obtain a water-based facial white.

The water-based facial white of the present invention has good dispersibility, a fresh feel, and good stability.

### (Example 61: Preparation of water-based eyeshadow)

A water-based eyeshadow of compositions shown in Table 23 was prepared.

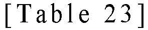

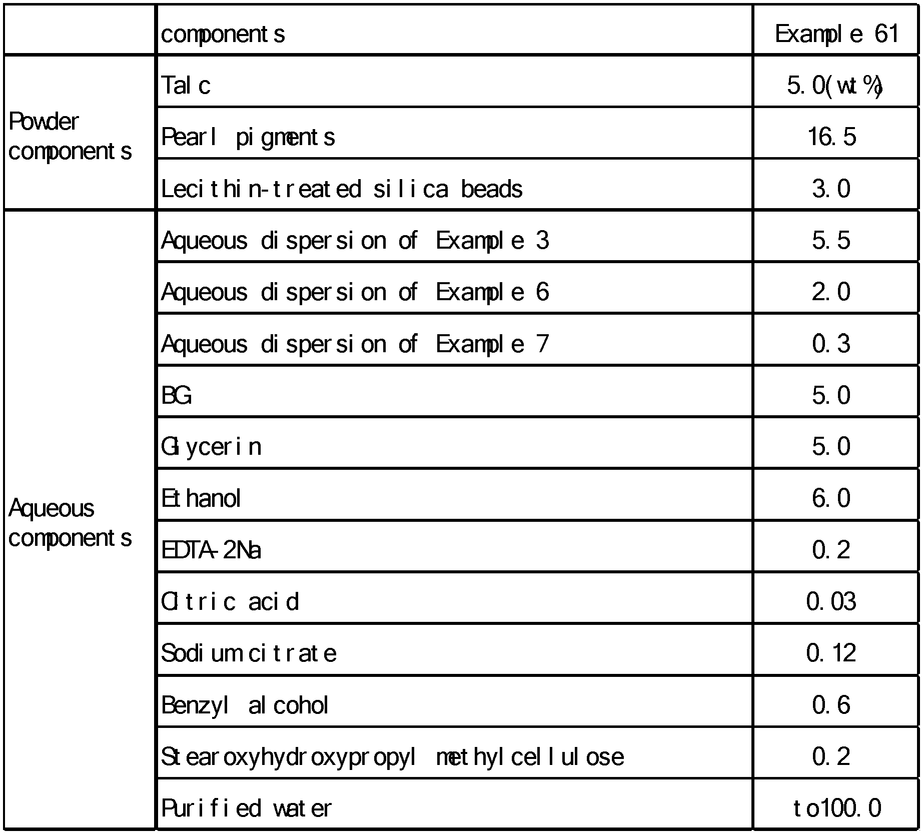

### (Preparation method)

A: Mix powder components well.
B: Mix and dissolve aqueous phase components.
C: Add A to B and stir well to obtain aqueous eyeshadow.

The water-based eyeshadow of the present invention had a good dispersibility of powder, good coloring, fresh feel, and good stability.

### (Example 62: Preparation of lipstick)

A lipstick of compositions shown in Table 24 was prepared.

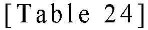

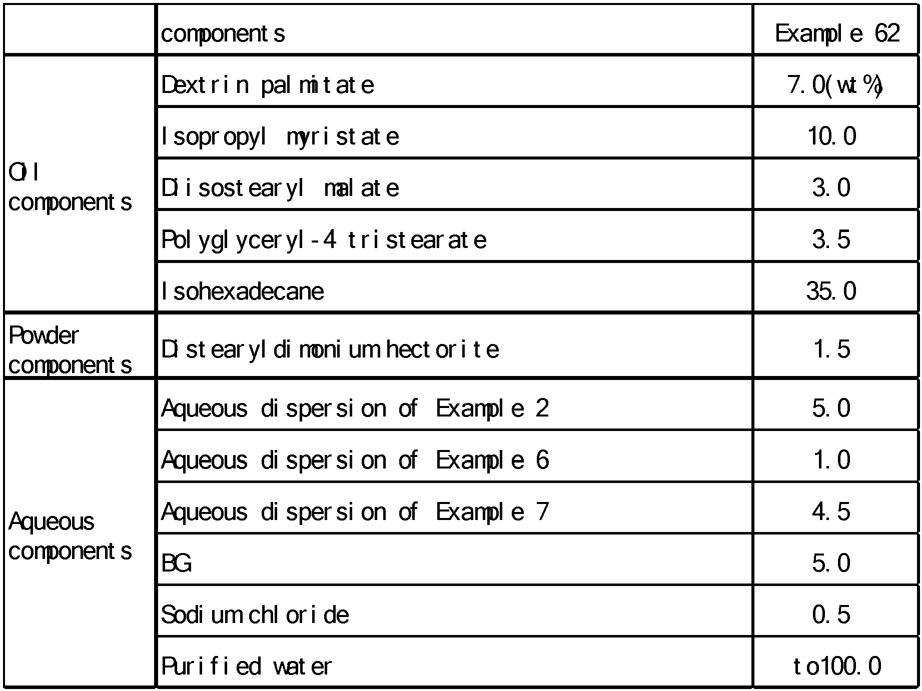

### (Preparation method)

A: Mix oil phase components well.
B: Mix powder components with component A and disperse it with a roller.
C: Add aqueous phase components to B and heat to be emulsified, to obtain a lipstick.

The lipstick of the present invention had a good dispersibility of powder, good coloring, fresh feel, and good stability.

### (Example 63: Preparation of antiperspirant)

An antiperspirant of compositions shown in Table 25 was prepared.

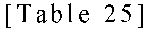

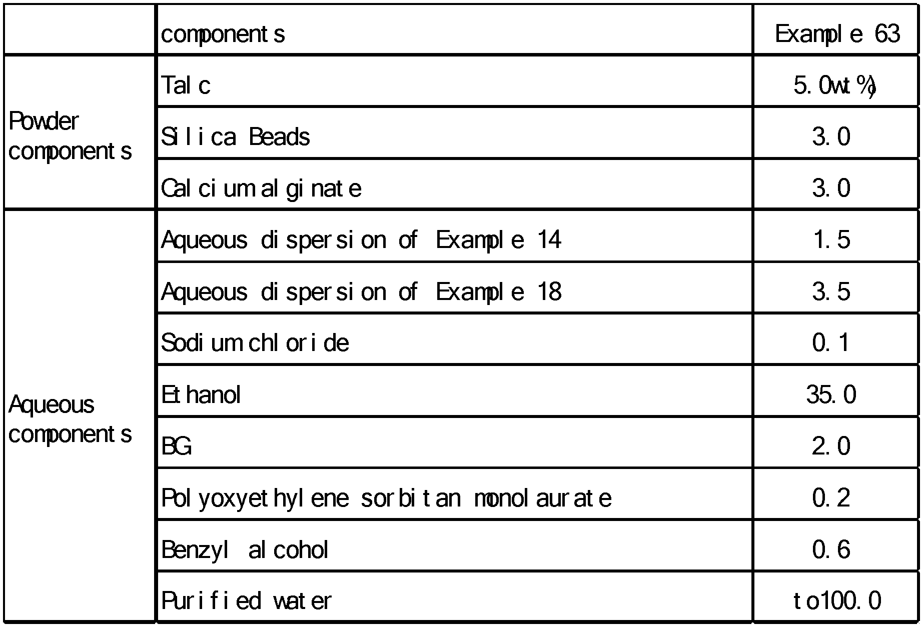

### (Preparation method)

A: Mix powder components well.
B: Mix and dissolve aqueous phase components.
C: Add A to B and mix it to obtain an antiperspirant.

The antiperspirant of the present invention had a good dispersibility of powder, fresh feel, and good stability.

### (Example 64: Preparation of powder foundation)

A powder foundation of compositions shown in Table 26 was prepared.

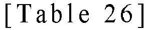

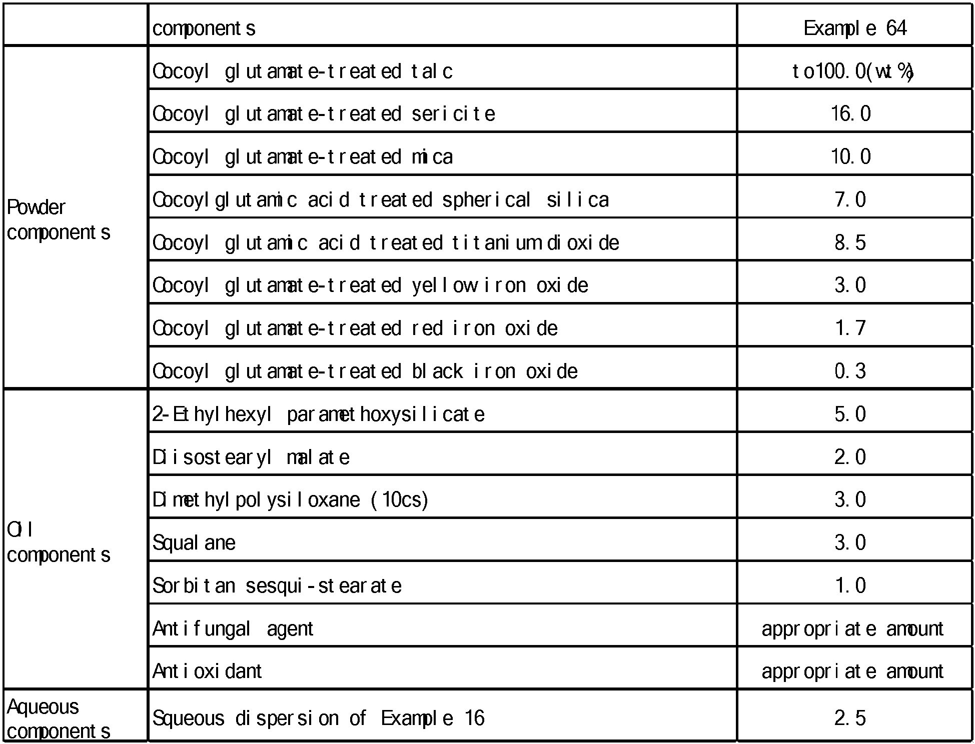

### (Preparation method)

A: Disperse and mix powder components well.
B: Mix and dissolve oil components well.
C: Add B to A and mix and pulverize it, then add aqueous components and mix and pulverize it.
D: Pass C through a screen and form in a metal plate to obtain a powder foundation.

The powder foundation of the present invention had a good dispersibility of powder, fresh feel, and good stability.

### (Example 65: Preparation of flow-in type powder foundation)

A powder foundation of compositions shown in Table 27 was prepared.

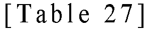

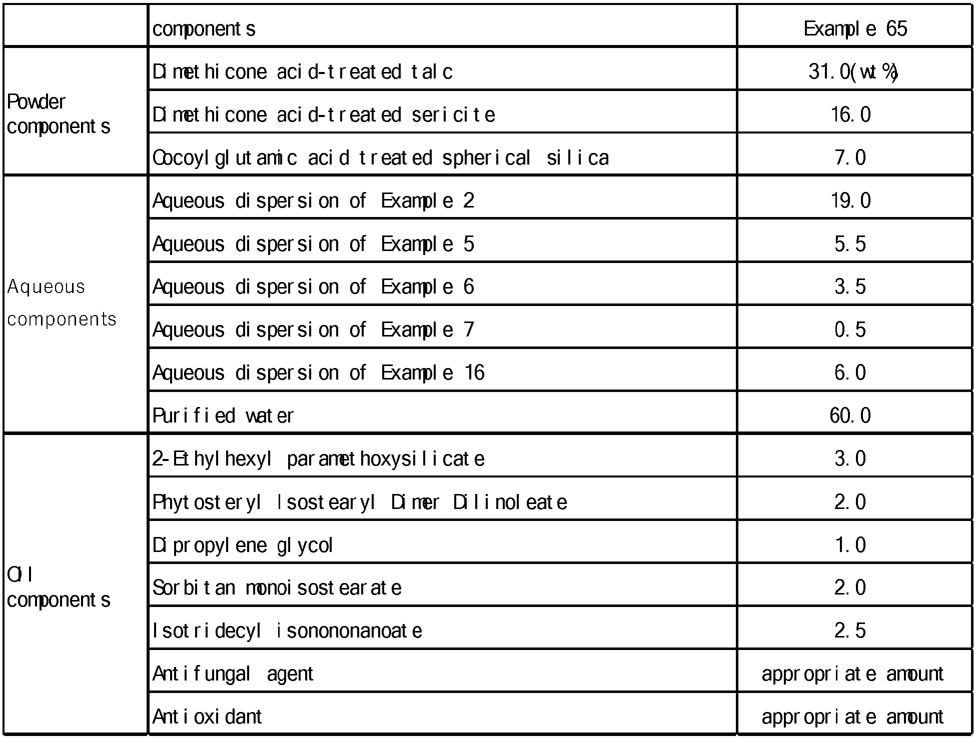

### (Preparation method)

A: Mix powder components well.
B: Mix A and oil components well.
C: Mix aqueous components well.
D: Add B to C and mix to form a slurry.
E: Charge D into a metal plate, place a water-absorbing sheet on its surface, and perform suction-compress molding using a porous suction head.
F: Place E in a thermostatic bath at 70°C for 24 hours to completely remove purified water to obtain a powder foundation.

The powder foundation of the present invention had a good moldability, good coloration, good feel and uniform finish, and good stability.

## Claims

1. An aqueous dispersion, comprising at least 3 components of
(A) polyoxyethylene (5 to 15) isostearyl ether,
(B) water, and
(C) hydrophobized inorganic powder,
wherein the aqueous dispersion has an excellent dispersion stability and excellent suitability in use satisfying a cycle test: -20°C to 40°C/2 weeks, a cycle test: 5°C to 60°C/2 weeks and an acceleration test 40± 2°C/75%RH ± 5%RH/6 months, and
(C) the hydrophobized inorganic powder comprises any one or more of titanium oxide, zinc oxide, or iron oxide having a primary particle size of 3µm or less, and wherein the aqueous dispersion has a content ratio of the hydrophobized inorganic powder of 40wt% or more.

2. The aqueous dispersion according to Claim 1, wherein
the hydrophobized inorganic powder as component (C) is an inorganic powder treated with one or more of hydrophobic agents: silicone compounds, fatty acids, acylated amino acids, hydrogenated lecithin, ester oils, alkyl silanes, and alkyl phosphates.

3. The aqueous dispersion according to Claim 1 or 2, wherein
the polyoxyethylene (5 to 15) isostearyl ether as the component (A) is polyoxyethylene (10) isostearyl ether.

4. A cosmetic, containing the aqueous dispersion according to any one of Claim 1 to 3.
